(19) Europäisches Patentamt
European Patent Office
Office européen des brevets

(11) Veröffentlichungsnummer: **0 279 273 B1**

(12) **EUROPÄISCHE PATENTSCHRIFT**

(45) Veröffentlichungstag der Patentschrift: **15.12.93**

(51) Int. Cl.[5]: **C12N 15/52**, C12N 1/20, C12P 17/12, //(C12N1/20, C12R1:19)

(21) Anmeldenummer: **88101442.7**

(22) Anmeldetag: **02.02.88**

# (54) DNA-Sequenzen, Plasmide und Mikroorganismen sowie Verfahren zur Herstellung von Chinolinsäure.

(30) Priorität: **04.02.87 DE 3703255**

(43) Veröffentlichungstag der Anmeldung: **24.08.88 Patentblatt 88/34**

(45) Bekanntmachung des Hinweises auf die Patenterteilung: **15.12.93 Patentblatt 93/50**

(84) Benannte Vertragsstaaten: **BE DE FR GB IT NL**

(56) Entgegenhaltungen:

**AGRIC. BIOL. CHEM., Band 47, Nr. 10, 1983, Seiten 2405-2408; M. KUWAHARA et al.: "Synthesis of quinolinic acid by the enzyme preparation of Escherichia coli which contains a plasmid carrying the nad gene for the de novo synthesis of NAD"**

(73) Patentinhaber: **RÜTGERSWERKE AKTIENGESELLSCHAFT**
**Mainzer Landstrasse 217**
**D-60326 Frankfurt(DE)**

(72) Erfinder: **Läufer, Albrecht, Dr.**
**Maybachstrasse 5**
**D-4300 Essen 1(DE)**
Erfinder: **Höke, Hartmut, Dr.**
**Erfurter Strasse 89**
**D-4620 Castrop-Rauxel(DE)**
Erfinder: **Höltmann, Wilhelm, Dr.**
**Dorffeldstrasse 9a**
**D-4400 Münster(DE)**
Erfinder: **Stadelhofer, Jürgen, Dr.**
**Falkenstrasse 85**
**D-6232 Bad Soden(DE)**
Erfinder: **Gassen, Hans-Günter, Prof. Dr.**
**Flachsbachweg 54**
**D-6100 Darmstadt(DE)**
Erfinder: **Flachmann, Ralf**
**Waldstrasse 78**
**D-6109 Mühltal/Traisa(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 279 273 B1

CHEMICAL ABSTRACTS, Band 79, Nr. 3, 23. Juli 1973, Seite 208, Zusammenfassung Nr. 15638u, Columbus, Ohio, US; N. SUZUKI et al.: "De novo biosynthesis of NAD in Escherichia coli. V. Properties of the quinolinic acid synthetase system"

CHEMICAL ABSTRACTS, Band 83, Nr. 3, 21. Juli 1975, Seite 251, Zusammenfassung Nr. 24848x, Columbus, Ohio, US; G.R. GRIFFITH et al.: "De novo biosynthesis of NAD in Escherichia coli. Separation of the nadB gene product from the nadA geneproduct and its purification"

EUR. J. BIOCHEM, Band 175, 1988, Seiten 221-228, FEBS; R. FLACHMANN et al.:"Molecular biology of pyridine nucleotide biosynthesis in Escherichia coli"

Erfinder: **Kunz, Norbert**
**Im Espenloh 28a**
**D-6087 Büttelborn(DE)**
Erfinder: **Seifert, Jochen**
**Beerfeldener Weg**
**D-6120 Michelstadt(DE)**

## Beschreibung

Die Erfindung betrifft ein Verfahren zur Herstellung von Chinolinsäure (Pyridin-2,3-dicarbonsäure) mit Hilfe gentechnisch modifizierter Mikroorganismen.

Chinolinsäure ist ein wichtiges Zwischenprodukt für zahlreiche Pharmazeutika und Pflanzenschutzmittel. Sie wird großtechnisch hergestellt durch Oxidationsverfahren von Chinolin oder Chinolinderivaten gemäß z. B. EP-B 82 542 oder EP-A 149 857. Ein Nachteil dieser Verfahren ist es, daß die verfügbare Rohstoffmenge nicht ausreicht, um den stetig wachsenden Bedarf an Chinolinsäure zu decken.

Es ist daher Aufgabe vorliegender Erfindung, ein Verfahren zur Herstellung von Chinolinsäure bereitzustellen, bei dem diese aus völlig anderen, unbegrenzt verfügbaren Rohstoffen in einem einfachen, kostengünstigen und umweltfreundlichen Verfahren gewonnen wird.

Die Lösung der Aufgabe besteht aus einem Herstellungsverfahren mit Hilfe gentechnisch modifizierter Mikroorganismen, aus der Bereitstellung und Herstellung solcher Mikroorganismen durch Isolierung und Bestimmung von DNA-Sequenzen, die für die Synthese der Enzyme Chinolinsäuresynthase und L-Aspartatoxidase codieren, deren Kombination mit Plasmid-DNA-Sequenzen und Vereinigung der so hergestellten rekombinanten Plasmide mit einem beliebigen Mikroorganismus gemäß der Ansprüche 1 bis 15.

Chinolinsäure ist ein natürliches intermediäres Stoffwechselprodukt vieler Organismen bei der Biosynthese des Nikotinamidadenindinukleotids (NAD). Als Zwischenprodukt tritt es allerdings in so geringen Konzentrationen auf, daß natürlich vorhandene Organismen für eine technische Herstellung keine Anwendung finden können.

Ist dagegen in einer sogenannten nadC-Mutante die Decarboxylierung der Chinolinsäure und die gleichzeitig ablaufende N-Phosphoribosylierung zur N-Phosphoribosylnikotinsäure blockiert, so lassen sich in einem nährstoffreichen Nährmedium bis zu 73 mg/l Chinolinsäure nachweisen (J.L.R. Chandler, R.K. Gholson, J. Bacteriol. 111 (1972) 96-102). Diese Konzentrationen sind für eine technische Gewinnung der Chinolinsäure aus Nährmedium zu gering.

Es ist daher notwendig, Organismen, insbesondere Mikroorganismen, so zu modifizieren, daß sie Chinolinsäure in einem Ausmaß produzieren, das für ein technisches Verfahren ausreichend ist.

Zur Modifizierung der Mikroorganismen werden erfindungsgemäß aus Mikroorganismen, die die Chinolinsäuresynthese als Teil des Stoffwechsels durchführen, DNA-Sequenzen gewonnen, die für die Enzyme Chinolinsäuresynthase (nadA) und L-Aspartatoxidase (nadB) codieren.

Hierbei steht die Enzymbezeichnung L-Aspartatoxidase für ein Enzym, das die folgende Reaktion katalysiert:

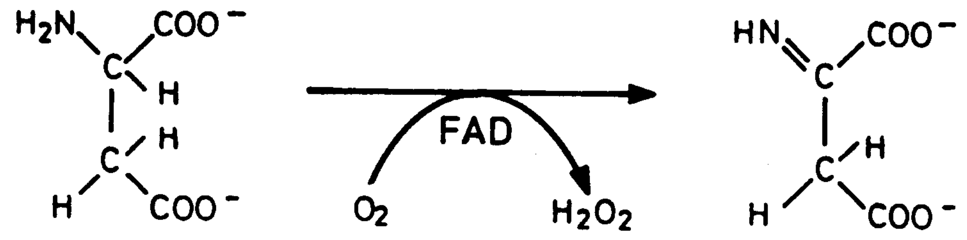


L-Aspartat    Iminoaspartat

Mit Chinolinsäuresynthase wird ein Enzym bezeichnet, das die folgende Reaktion katalysiert:

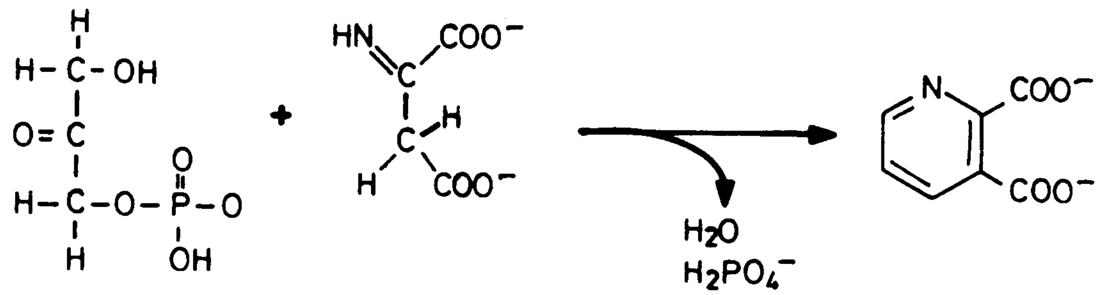


Dihydroxyaceton-phosphat (DHAP) Iminoaspartat Chinolinat

DNA-Sequenzen, die für die genannten Enzyme codieren, sind in der genomischen Information vieler Mikroorganismen vorhanden, wie z. B. in

Escherichia coli
Salmonella typhimurium
Mycobacterium tuberculosis
Mycobacterium bovis
Bacillus subtilis
Saccharomyces cerevisiae.

Diese Mikroorganismen sind für die Isolierung von nadA und nadB geeignet. In der Literatur sind nadA- und nadB-Mutanten von E.coli K12 beschrieben.

Die Isolierung und Insertion eines Gens für die de novo Chinolinsäure-Biosynthese (vermutlich nadB) aus chromosomaler E.coli DNA in das Plasmid pBR322 zu einem Plasmid pNADH1 wird bei Kuwahara et al. (M. Kuwahara, M. Yonehana, T. Kimura und Y. Ishida, Agric. Biol. Chem. 47 (1983) 2405-8) beschrieben. Zellfreie Extrakte aus pNADH1-Plasmid-haltigen E.coli C600-Zellen sollen eine um das ca. fünffache erhöhte Chinolinsäuresynthese aufweisen, wenn man von Dihydroxyacetonphosphat und L-Asparaginsäure als Vorstufen ausgeht.

Hingegen wurden aber weder Chinolinsäuresynthese noch Chinolinsäureausscheidung in ruhenden oder wachsenden Zellen, die durch plasmidgebundene Gene bewirkt wird, gezeigt. Nur so ist jedoch die Verwendung billiger Kohlenstoff- und Stickstoffquellen möglich.

In der Literatur wird verschiedentlich diskutiert, daß für die Chinolinsäuresynthese aus L-Aspartat bis zu sechs Enzyme notwendig sind. Es wurde gefunden, daß lediglich die beiden Enzyme L-Aspartatoxidase und Chinolinsäuresynthase für eine gesteigerte Chinolinsäuresynthese ausreichen.

Es wurde weiterhin gefunden, daß zur Lösung der erfindungsgemäßen Aufgabe Plasmide konstruiert werden müssen, die beide DNA-Sequenzen nadA und nadB enthalten.

Da diese beiden Gene in den Mikroorganismen genomisch getrennt kartiert sind, mußten Plasmide konstruiert werden, die die zwei DNA-Sequenzen, die aus genomisch getrennten Genen bestehen und die jeweils für ein Enzym des Stoffwechsels codieren, enthalten und gemeinsam wirksam werden lassen.

Dazu wurden folgende Schritte durchgeführt:

- Identifizierung und Isolierung der DNA-Sequenz nadB, die für das Enzym L-Aspartatoxidase codiert.
- Identifizierung und Isolierung der DNA-Sequenz nadA, die für das Enzym Chinolinsäuresynthase codiert.
- Einbau der DNA-Sequenzen nadA und nadB in Plasmide, so daß jede DNA-Sequenz mit einer Expressions-Kontroll-Sequenz verbunden ist.
- Einbau der Strukturgen-Sequenzen der DNA-Sequenzen nadA und nadB hintereinander unter gemeinsamer Kontrolle einer einzigen Expressions-Kontroll-Sequenz.
- Einbringen der so hergestellten modifizierten Plasmide in einen transformierbaren Wirtsorganismus.

Die so hergestellten neuen Mikroorganismen produzieren in einem Nährmedium mit organischer C-Quelle und anorganischer oder organischer N-Quelle wesentlich mehr Chinolinsäure als es dem Stand der Technik entspricht. Die produzierte Chinolinsäure wird nicht mehr dem ursprünglichen Metabolismus gemäß umgesetzt, sondern bevorzugt ausgeschieden. Somit ist eine technische Gewinnung von Chinolinsäure möglich.

Zur Isolierung der DNA-Sequenz nadB wird aus einem geeigneten Mikroorganismus chromosomale DNA isoliert. Diese DNA wird mit der Restriktionsendonuclease HindIII hydrolysiert. Die entstandenen Fragmente werden auf einem Polyacrylamidgel elektrophoretisch aufgetrennt.

Aus dem Gel werden die zwischen 6 und 8 kbp liegenden DNA-Fragmente elektroeluiert und mit dem ebenfalls mit HindIII linearisierten Vektor pBR322 ligiert. Die Selektion auf nadB-Gen erfolgt durch Transformation in eine nadB-Mutante (Eine nadB-Mutante enthält keine L-Aspartatoxidase (nadB-Enzym) und ist daher nicht in der Lage, die Oxidation von L-Aspartat zu Iminoaspartat durchzuführen.) und Test auf Komplementation auf einem Minimalmedium (z. B. R.A. Yates und A.B. Pardee, J.Biol.Chem. 221 (1956) 643-756). Es wird ein ca. 13 kbp großes, nadB-Mangel-komplementierendes Plasmid pCH100 isoliert.

Die Restriktionsanalyse ergibt u. a. eine NruI-Schnittstelle auf dem Insert, die zur Subklonierung verwendet wird. Ein ca. 3.2 kbp großes NruI-HindIII-Fragment wird in mit HindIII und NruI hydrolisierten pBR322 zu einem neuen Plasmid pCH101 inseriert. Die Plasmide pCH100 und pCH101 ergeben in wiederholten Retransformationsversuchen in eine nadB-Mutante Komplementation des nadB-Mangels. Weitere Subklonierungen ergaben ein 1,6 kbp großes Fragment (SspI-AccI) das in in einer nadB-Mutante den nadB-Mangel komplementiert.

Von diesem Fragment wird die Nukleotidsequenz ermittelt.

Parallel zu der Isolierung des nadB-Gens wird mit Hilfe protein-biochemischer Techniken das nadB-Enzym, die L-Aspartatoxidase aus E.coli-Mutanten rein dargestellt (hierfür wird ein Enzym-Aktivitätstest neu erstellt) und mittels automatisiertem Edman-Abbau N-terminal sequenziert.

Ein Vergleich der N-terminalen Proteinsequenz und der Nukleotidsequenz ergibt den Startpunkt des nadB-Strukturgens, ca. 450 bp von der HindIII-Schnittstelle entfernt.

Zur Isolierung der DNA-Sequenz nadA wird ebenfalls aus einem geeigneten Mikroorganismus chromosomale DNA isoliert und mit der Restriktionsendonuclease Sau3A hydrolysiert; die entstandenen DNA-Fragmente werden in die BamHI-Schnittstelle des Plasmids pBR322 inseriert und in eine nadA-Mutante transformiert.

Dabei wurde überraschenderweise gefunden, daß Insertion in das üblicherweise verwendete high copy Plasmid pBR322 für die transformierte Wirtszelle lethal ist. Erst die Verwendung eines low copy Plasmids erlaubte die Isolierung einer nadA-DNA-Sequenz; die Selektion des nadA-Gen tragenden Plasmids erfolgt durch Komplementation nach Transformation in eine nadA-Mutante auf Minimalmedium nach an sich bekannten Verfahren.

Das isolierte Plasmid pCH200 enthält ein ca. 12 kbp großes Insert. Die Retransformation in nadA-Mutante 431 ergibt Komplementation des nadA-Mangels.

Partielle Hydrolyse mit der Restriktionsendonuclease HaeIII und Klonierung in die HincII-Schnittstelle des Vektors pUC18 ergibt das Plasmid pCH201 mit einem ca. 1.4 kbp großen Insert. Die Nukleotidsequenz dieses 1.4 kbp Inserts wird vollständig beidsträngig ermittelt und ergibt neben dem Strukturgen mit seiner Start- und Stop-Sequenz auch die Promotorregion.

Erfindungsgemäß sind die DNA-Sequenzen nadA und nadB bereits mit je einer Expressions-Kontroll-Sequenz verbunden. Sie haben als Expressions-Kontroll-Sequenz die genomischen Regulationssequenzen. Sie können jedoch auch mit Expressions-Kontroll-Sequenzen verbunden werden, die an sich bereits bekannt sind. (Z. B.: H. Bujard, U. Deuschle, W. Kammerer, R. Creutz, W. Bannwarth, D. Stueber, UCLA Symp. Mol. Cell. Biol., New Ser. 1985, 30, 21-29; E. Remant, P. Stanssens, F. Fiers, Gene 15 (1981) 81-93).

Dazu gehören:

- der E.coli trp-Promotor,
- der E.coli tac-Promotor,
- der E.coli beta-Lactamasepromotor,
- der E.coli Lipoproteinpromotor,
- eine Hefe-Expressions-Kontroll-Sequenz,
- eine Pseudomonaden-Expressions-Kontroll-Sequenz oder eine andere prokaryontische Expressions-Kontroll-Sequenz.

Wichtig ist jeweils die funktionelle Verknüpfung des Gens mit der Expressions-Kontroll-Sequenz sowie die Auswahl einer geeigneten Expressions-Kontroll-Sequenz für einen bestimmten Wirtsorganismus. In einer weiteren Ausführung der Erfindung sind die beiden DNA-Sequenzen nadA und nadB hintereinandergeschaltet und haben gemeinsam eine Expressions-Kontroll-Sequenz.

Diese mitunter als rekombinante Expressionsplasmide benannten Expressionsvektoren werden erfindungsgemäß als Plasmide bezeichnet. Sie werden in an sich bekannter Weise erhalten durch Ligation der einzelnen DNA-Fragmente und Insertion der DNA-Fragmente in Plasmide. Im Gegensatz zu den Erkenntnissen bei der Isolierung der nadA-Sequenz läßt sich nun überraschenderweise die Insertion in ein high copy Plasmid durchführen.

Die erhaltenen Plasmide werden in üblicher Weise in einen transformierbaren Wirtsorganismus, z. B. ein E.coli-Bakterium, eingebracht. Anschließend werden die mit einem oder mehreren Plasmiden transformierten Mikroorganismen in an sich bekannter Weise in einem geeigneten Nährmedium kultiviert und das oder die bei der Expression gebildete(n) Polypeptid(e) mit der biologischen Aktivität des/der Enzyms/Enzyme L-Aspartatoxidase und/oder Chinolinsäuresynthase können daraus nach Standardmethoden isoliert und nach den entwickelten Tests nachgewiesen werden.

Die Produktion und Gewinnung von Chinolinsäure erfolgt in an sich bekannter Weise durch die mit der erfindungsgemäßen DNA transformierten Mikroorganismen unter geeigneten Bedingungen, wie Batch-, Fed-batch- oder kontinuierliche Fermentation in Rühr- oder Airlift oder ähnlichen Bioreaktoren, wobei einem erfindungsgemäßen Mikroorganismus in einem Nährmedium eine organische Kohlenstoffquelle und eine anorganische oder organische Stickstoffquelle unter Wachstumsbedingungen zur Verfügung gestellt werden.

Die Erfindung wird durch die folgenden Abbildungen und Beispiele näher erläutert.

Liste der Abbildungen:

- Abb. 1 Restriktionskarte des 3.2 kbp-HindIII/NruI-Inserts in pCH101
- Abb. 2
  - a. Sequenzierungsstrategie nadB-Gen
  - b. Nukleotidsequenz des nadB-Gens aus E.coli K12
- Abb. 3 Restriktionskarte des 1.4 kbp-HaeIII-Inserts in pCH201
- Abb. 4
  - a. Sequenzierungsstrategie nadA-Gen
  - b. Nukleotidsequenz des nadA-Gens aus E.coli K12
- Abb. 5 Restriktionskarte des Plasmids pCH400

Verwendete Abkürzungen:

| | |
|---|---|
| Ap | Ampicillin |
| Bicine | Bishydroxymethylglycin |
| BFM | Biofeuchtmasse |
| BTM | Biotrockenmasse |
| BSA | Rinderserumalbumin |
| bp | Basenpaare |
| DHAP | Dihydroxyacetonphosphat |
| DMSO | Dimethylsulfoxid |
| DNA | Desoxyribonukleinsäure |
| DTT | Dithiothreitol |
| E.coli | Escherichia coli |
| EDTA | Ethylendiamintetraessigsäure |
| FAD | Flavin-Adenin-Dinukleotid |
| FPLC | Fast Protein Liquid Chromatography |
| HPLC | High Performance Liquid Chromatography |
| kbp | Kilobasenpaare |
| Km | Kanamycin |
| LB | Luria-Bertani |
| PAGE | Poly-Acrylamid-Gel-Elektrophorese |
| RNA | Ribonukleinsäure |
| SDS | Sodium-Dodecyl-Sulfat |
| Tc | Tetracyclin |
| Tris | Trishydroxymethylglycin |
| UV | Ultraviolett |
| YP | Yates-Pardee |

Zusammensetzung der verwendeten Lösungen:

| | |
|---|---|
| TE | 10 mmol/l Tris, pH 8.0, 1 mmol/l EDTA |
| Lysozym-Puffer | 0.1 ml NaCl (5 mol/l)<br>1 ml EDTA (0.5 mol/l, pH 8.0)<br>0.3 ml Tris-Pufferkonzentrat (Tris-HCl) (1 mol/l) pH 8.0<br>8.6 ml $H_2O$ |
| Proteinase K Puffer | 0.01 mol/l Tris (Tris-HCl) pH 8.0<br>0.005 mol/l EDTA<br>0.5 % SDS |
| YP-Medium | 7 g $K_2HPO_4$<br>2 g $KH_2PO_4$<br>0.5 g Natriumcitrat x 5 $H_2O$<br>0.1 g $MgSO_4$ x 7 $H_2O$<br>1 g $(NH_4)_2SO_4$<br>2.5 g Glucose<br>2 mg Thiamin<br>ad 1000 ml $H_2O$ |
| A-Medium | YP-Medium<br>+ Nikotinsäure ($10^{-6}$ mol/l)<br>+ L-Aspartat (5 x $10^{-6}$ mol/l)<br>+ Caseinhydrolysat (5 g/l)<br>5 g/l Glycerin statt 2.5 g Glucose |
| LB-Medium | 10 g Caseinhydrolysat oder Pepton<br>5 g Hefeextrakt<br>5 g NaCl<br>ad 1000 ml $H_2O$, pH 7.4 |

Bei Einsatz von Antibiotika wurden folgende Konzentrationen angewandt:
Ampicillin (Ap): 100 mg/l
Tetracyclin (Tc): 2 mg/l
Kanamycin (Km): 25 mg/l

Beispiel 1

Präparation chromosomaler DNA aus Escherichia coli

100 ml Nährlösung (LB-Medium) werden mit 1 ml einer Vorkultur von E.coli K12 C600 in der gleichen Nährlösung beimpft und 18 h bei 37 °C inkubiert. Die Zellsuspension wird in auf Eis gestellte Zentrifugenröhrchen verteilt und 10 min bei 4 °C und 2600 x g zentrifugiert.

Die sedimentierten Zellen werden in 10 ml Lysozym-Puffer suspendiert und mit 40 mg Lysozym 30 min bei 37 °C inkubiert. Die Suspension wird auf 1 % SDS eingestellt und 30 min bei 37 °C inkubiert. Nach Zusatz von 5 mg Proteinase K (Merck) wird 3 h bei 50 °C inkubiert. Danach wird vorsichtig viermal mit TE-gesättigtem Phenol (TE) und anschließend dreimal mit Chloroform/Isoamylalkohol (24 : 1) extrahiert.

Anschließend wird die wäßrige Phase auf eine Konzentration von 0.3 mol/l an Natriumacetat eingestellt und die DNA durch Zugabe des doppelten Volumens an Ethanol gefällt. Die DNA wird abzentrifugiert, im Exsikkator getrocknet und danach in 5 ml TE-Puffer gelöst. Dann werden 50 mikro g DNase-freie Ribonuclease A (Sigma) zugegeben (Inaktivierung der DNase durch vorheriges Kochen der RNase in 100 mM Natriumacetat bei pH 5.5 für 10 min). Nach 30minütiger Inkubation Zugabe von SDS auf 1 % Endkonzentration und Zugabe von 250 mikro g Proteinase K werden weitere 30 min bei 37 °C inkubiert.

Danach wird vorsichtig viermal mit TE-gesättigtem Phenol und zehnmal mit Chloroform/Isoamylalkohol (24:1) extrahiert. Die Lösung wird zweimal 12 h gegen 1000 ml TE dialysiert und bei 4 °C aufbewahrt.

Beispiel 2

Klonierung genomischer Sequenzen: Material und Methoden

Enzyme: Restriktionsendonucleasen, DNA-Ligase des Phagen T4, Klenov-Fragment der E.coli DNA-Polymerase Pol I, Reverse Transscriptase (MMLV) wurden bei Gibco-BRL, alkalische Phosphatase, Ribonuclease A und Lysozym bei Boehringer (Mannheim) und Proteinase K bei Merck gekauft und nach den Angaben der Hersteller verwendet.

E.coli-Stämme PA2-18 (CGSC Nr. 5176), NK6033 (CGSC Nr. 6180), NK6042 (CGSC Nr. 6184), W4546 (CGSC Nr. 5179), C600 (CGSC Nr. 3004) wurden vom E.coli Genetic Stock Center (CGSC), die Stämme JM101, JM103, JM109, DH5 von Gibco BRL, der Stamm 431 von Dr. B. Rak (Freiburg), der Stamm GE 1806 von Dr. W. Schumann (Darmstadt), bezogen.

Plasmide (pBR322, pUC18, pUC19, pLG339) wurden bei Pharmacia, das Plasmid pLG339 (N.G. Stoker et al., Gene 18 (1982) 335-341) von den Autoren bezogen, in geeigneten Wirtszellen propagiert und nach folgendem Verfahren präpariert:
1000 ml LB-Medium werden mit 3 ml einer Übernachtkultur des plasmidtragenden Wirtsstammes beimpft und 18 h bei 37 °C inkubiert.

Die Zellsuspension wird 15 min bei 2600 x g und 4 °C zentrifugiert. Die sedimentierten Zellen werden in insgesamt 20 ml 20 % Saccharose, 10 mM Tris-HCl, pH 8.0, suspendiert und 10 min auf Eis inkubiert. Danach werden 3 ml 0.5 M EDTA, pH 8.0, und 3 ml 20 mg/ml Lysozymlösung zugesetzt und damit 45 min auf Eis inkubiert. Die Lösung wird auf 2 Zentrifugenröhrchen aufgeteilt.

Danach werden 2.6 ml einer Lösung, die 5 % Brij58 (Polyoxyethylenemonocetylether) und 2 % Desoxycholat enthält, zugesetzt, und es wird exakt 5 min auf Eis inkubiert. Anschließend wird bei 60000 x g und 4 °C für 25 min zentrifugiert. Nach Zugabe von Natriumacetat auf Endkonzentration von 0.3 mol/l wird die DNA mit dem doppelten Volumen Ethanol bei - 70 °C gefällt.

Die DNA wird bei 10000 x g und 4 °C 20 min abzentrifugiert, im Vacuum getrocknet und in 10 ml Proteinase K Puffer gelöst. Nach Zugabe von 1 mg Proteinase K wird bei 50 °C 60 min inkubiert. Anschließend wird je dreimal mit TE-gesättigtem Phenol und Chloroform/Isoamylalkohol (24 : 1) und einmal mit Diethylether extrahiert.

Nach Einstellen der wäßrigen Phase auf 0.3 mol/l Natriumacetat wird die DNA mit dem doppelten Volumen Ethanol bei - 70 °C gefällt, bei 10000 x g und 4 °C 30 min abzentrifugiert und im Vakuum getrocknet.

Der Niederschlag wird in 29 ml TE, die 28.14 g CsCl enthalten, gelöst. Die Lösung wird mit 2.9 ml Ethidiumbromid-Lösung versetzt (10 mg/ml), in ein 39 ml fassendes Quick-seal-Zentrifugenröhrchen (Beckman Instruments) gefüllt, und es wird im Vertikalrotor bei 160000 x g und 15 °C 16 h zentrifugiert. Unter UV-Licht wird die "Covalently Closed Circular"-DNA-Bande abgezogen; Ethidiumbromid wird mit TE-gesättigtem Butanol extrahiert, Butanol und CsCl werden durch Dialyse (24 h, gegen 2 x 1 l TE) entfernt.

Die DNA( = Plasmid)-Konzentration in der wäßrigen Lösung wird durch Messung der UV-Absorption bei 260 nm bestimmt.

Plasmid-DNA, die zur Sequenzierung eingesetzt werden soll, wird durch Fällung mit Spermin (B.C. Hoopes, W.R. McClure, Nucl. Acid. Res. 9 (1981) 5493-5504) zusätzlich gereinigt.

Durch restriktionsenzymatische Hydrolyse erhaltene DNA-Fragmente werden auf Polyacrylamid-Gel aufgetrennt (T. Maniatis, F. Fritsch, J. Sambrock; Molecular Cloning, A Laboratory Manual; Cold Spring Harbor Laboratory 1982, S. 150 ff.); Fragmentelution erfolgt durch Elektroelution (Maniatis S. 164).

Die Transformation von E.coli-Stämmen erfolgt nach der $CaCl_2$-Methode (M. Mandel, A. Higa, Mol. Biol. 53 (1970) 154).

Der Phage T4GT7 (G. Wilson, K.K.V. Young, G.J. Edlin, Nature 280 (1979) 80-81) wurde von den in der Literaturstelle angegebenen Autoren bezogen.

Transduktion: Es wird das Prinzip der "generalisierten" Transduktion (R.E. Glass, Gene Function, Croom Helm London (1982), S. 210 ff.) mit Hilfe des Phagen T4GT7 zur Überführung genomischer DNA eines Donorstammes in einen Rezeptorstamm angewandt.

Experimentelles Vorgehen:
Die Gewinnung von Phagenlysat, die Titerbestimmung und Infektion der Wirtszellen erfolgt nach der an sich bekannten Methode (Maniatis).

Im Einzelnen werden 100 mikro l T4GT7-Lysat vermischt und 15 min bei 37 °C inkubiert. Hierbei werden die Zellen des Donorstammes vom Phagen infiziert; während des Vermehrungs- und Lysezyklus werden mit geringer Wahrscheinlichkeit Fragmente genomischer DNA anstelle der Phagengenome in Phagenhüllen eingekapselt.

Zur Gewinnung des Phagenlysats, das nun auch eingekapselte genomische DNA-Fragmente enthält, wird die Weichagarschicht nach Zugabe von 1 ml LB-Medium von der Agarplatte abpipettiert. Die so gewonnene Suspension wird mit 0,5 ml $CHCl_3$ ausgeschüttelt und zentrifugiert (15 min, 8000 x g, 20 °C).

Der phagenhaltige wäßrige Überstand wird als Phagenlysat bezeichnet. Es wird eine Titerbestimmung nach an sich bekannten Methoden mit dem Stamm C600 durchgeführt.

Zur Transduktion der Donor-DNA in den Rezeptorstamm werden je 100 mikro l einer Übernachtkultur des Rezeptorstammes mit 5, 10, 50 und 100 mikro l Phagenlysat und 195, 190, 150 und 100 mikro l LB-Medium vermischt und nach 15 min Inkubation bei 37 °C mit je 2,5 ml LB-Weichagar (0,7 % Agar) auf Agarplatten verteilt und 16 h bei 37 °C inkubiert.

Die Identifikation der Zellkolonien, die das gewünschte transduzierte Gen enthalten, erfolgt durch ein geeignetes Selektionsmedium, das in den Agarplatten eingesetzt wird (z. B. Zusatz von Tetracyclin bei der Identifikation der erfolgreichen Transduktion eines Transposon Tn10-tragenden Genes; Tn10 bewirkt Tetracyclinresistenz).

Zur Bestimmung der Nukleotidsequenzen werden mit Hilfe von Restriktionsendonucleasen DNA-Fragmente geeigneter Länge (zwischen 100 und 400 bp) hergestellt und in passende Restriktionsschnittstellen der Plasmide pUC18 oder pUC19 kloniert. Die Sequenzierung erfolgt nach dem Kettenabbruchverfahren ("Dideoxy-Methode") nach Sanger (F. Sanger, S. Micklen, A. R. Coulson, Proc. Natl. Acad. Sci. USA 74 (1977) 5463-5467) unter Verwendung der Arbeitsvorschrift "Plasmid Sequenzieren" (P. Heinrich, Inst. f. Biochemie der Ludwig-Maximilian-Universität, 8000 München, 1985).

## Beispiel 3

### Klonierung genomischer nadB-Sequenzen

30 mikro g nach Beispiel 1 isolierter chromosomaler DNA werden 18 h bei 50 °C mit der Restriktionsendonuclease HindIII hydrolysiert und anschließend auf einem 3.5 % Polyacrylamidgel in TBE-Puffer 20 h bei 30 mA elektrophoretisch aufgetrennt.

Die DNA-Fragmente mit Größen zwischen 6 und 8 kbp wurden aus dem Gel elektroeluiert, über eine Anionenaustauschersäule (DE52 Diethylaminoethyl-Cellulose, Whatman) gereinigt und mit Ethanol gefällt. 1 mikro g Fragment-DNA und 1 mikro g HindIII-hydrolysierte und dephosphorylierte pBR322-DNA werden in 20 mikro l Ligasepuffer (von Gibco-BRL) mit 10 Units T4-DNA-Ligase zur Ligation eingesetzt.

Transformation in die nadB-Mutante E.coli NK6042 (nadB::Tn10) und Selektion auf YP/Ap-Medium auf Komplementation des nadB-Mangels ergibt 7 Klone, die alle ein Plasmid der ungefähren Größe 13 kbp enthalten. Wiederholte Retransformation dieses Plasmids in NK6042 bestätigen die Komplementation des nadB-Mangels. Das Plasmid wird mit pCH100 bezeichnet und wie bei Maniatis beschrieben auf Restriktionsschnittstellen analysiert.

Subklonierung eines ca. 3.2 kbp großen HindIII-NruI-Fragments in mit HindIII und NruI hydrolysierten pBR322 ergibt ein weiteres Plasmid pCH101, welches nadB-Mangel in NK6042 komplementiert; von dem 3.2 kbp-Insert in pCH101 wird eine Restriktionskarte erstellt (Abb. 1).

## Beispiel 4

### Bestimmung der Nukleotidsequenz des nadB-Gens

Ausgehend von dem Plasmid pCH101 wurden Subklone in geeignete Restriktionsnucleaseschnittstellen der Polylinkersequenz der Plasmide pUC18 und pUC19 hergestellt. Es wurde gefunden, daß das ca. 1,6 kbp große SspI-AccI-Fragment (Abb. 2 a) das kleinste Fragment ist, welches nadB Mangel in NK6042 komplementiert. Dieses Fragment wurde nach Sanger vollständig und beidstrangig sequenziert.

Die Sequenzierungsstrategie ist in Abb. 2 a zusammengefaßt.

Die Nukleotidsequenz des nadB-Gens ist in Abb. 2 b gezeigt.

Beispiel 5

Isolierung, Reinigung und partielle Sequenzierung des nadB-Genprodukts mit der biologischen Aktivität der L-Aspartatoxidase

a. Analytischer Nachweis der L-Aspartatoxidase-Aktivität

Dem Nachweis der L-Aspartatoxidase-Aktivität liegt zugrunde, daß das durch Oxidation von L-Asparaginsäure mit L-Aspartatoxidase in Gegenwart von Sauerstoff und FAD intermediär entstehende Iminoaspartat in Abwesenheit von Chinolinsäuresynthase und Dihydroxyacetonphosphat spontan zu Oxalacetat und $NH_4^+$ hydrolysiert wird.

Die Menge gebildeten Oxalacetats ist damit der Aktivität der L-Aspartatoxidase proportional.

Oxalacetat wird modifiziert nach Bergmeyer (H. U. Bergmeyer, Methods of Enzymatic Analysis, Vol. 9, 1985) durch NADH-abhängige Reduktion zu Malat mit Malatdehydrogenase photometrisch bestimmt.

Durchführung:

1. Umsetzung von L-Aspartat zu Oxalacetat
Testansatz 285 mikro l $H_2O$
Testansatz 50 mikro l Bicine 1 mol/l, pH 8.0
Testansatz 50 mikro l BSA 10 mg/ml in wäßriger Lösung
Testansatz 5 mikro l EDTA 0.5 mol/l, pH 8.0
Testansatz 5 mikro l FAD 2 mmol/l
Testansatz 5 mikro l Katalase 5000 Units/ml
Testansatz 50 mikro l L-Aspartat 0.1 mol/l, pH 8.0
Testansatz 50 mikro l Proteinlösung (Probe)
Der Testansatz wird bei 37 °C für 30 min inkubiert; danach wird die Reaktion durch Zugabe von 200 mikro l $HClO_4$ (1.7 mol/l) beendet; die ausgefällten Proteine werden abzentrifugiert, der Überstand wird durch Zugabe von 75 mikro l KOH (5 mol/l) neutralisiert; das hier ausfallende $KClO_4$ wird durch Zentrifugation abgetrennt. Die Oxalacetatkonzentration im Überstand wird wie folgt bestimmt:
2. Bestimmung der Oxalacetatkonzentration
Testansatz 200 mikro l Bicine 1 mol/l, pH 8.0
Testansatz 5 mikro l EDTA 0.5 mol/l
Testansatz 585 mikro l Überstand aus 1.
Testansatz 100 mikro l NADH 1 mmol/l
Der Testansatz wird in einer Küvette durchgeführt; nachdem die Absorption bei 340 nm konstant ist, werden 10 mikro l Malatdehydrogenase (1 Unit/mikro l) zugegeben, und es wird die Extinktionsdifferenz bei 340 nm bestimmt. Der molare Extinktionskoeffizient von NADH bei 340 nm beträgt 6220 $dm^2$/mol. Eine Unit L-Aspartatoxidase ist diejenige Menge Enzym, die die Oxidation von einem mikro mol L-Aspartat in einer Minute katalysiert. Ergebnisse sind in Tabelle 1 niedergelegt.

b. Isolierung und Reinigung

Als Ausgangsstamm zur Isolierung des nadB-Genprodukts, im folgenden nadB-Enzym oder L-Aspartatoxidase genannt, wird eine mit dem Plasmid pCH101 transformierte nadA-Mutante (E.coli K12 PA2-18) verwendet.

Je 1 l LB-Medium werden mit je 5 ml Vorkultur (PA2-18/pCH101) beimpft und bei 37 °C 2 Tage geschüttelt. Durch Zentrifugation bei 4 °C werden insgesamt 35 g BFM gewonnen, die in 200 ml 50 mM $K_2HPO_4$-Puffer (pH 8.0, 0,1 mM DTT) aufgenommen werden. Mit einem Bronson Sonifier B15, eingestellt auf Stufe 10, werden die Zellen durch Ultraschallbehandlung für 30 min bei 10 - 15 °C aufgeschlossen. Nach Zentrifugation bei 10000 x g und 4 °C für 35 min werden ca. 220 ml Überstand mit ca. 4,3 g Protein erhalten (die Proteinbestimmung erfolgt durch Absorptionsmessungen bei 280 nm); dieser Überstand wird auf 25 % an Glycerin eingestellt.

Anschließend werden 43 ml einer 2%igen Protaminsulfatlösung bei 4 °C unter Rühren während 1 h langsam zugesetzt. Zentrifugation bei 28000 x g und 4 °C für 30 min ergibt einen Überstand (320 ml) mit ca. 1,7 g Protein. Nach Einstellen der Lösung auf 50 % an $(NH_4)_2SO_4$ wird bei 4 °C 4 h lang gerührt.

Nach Zentrifugation bei 16000 x g und 4 °C für 40 min wird der Niederschlag in 40 ml 50 mM Kaliumphosphat pH 8.0, 25 % Glycerin, 0,1 mM DTT gelöst und gegen 2 x 1 l (50 mM Kaliumphosphat pH

8.0, 25 % Glycerin, 0.1 mM DTT) 16 h dialysiert.

Zentrifugation bei 17500 x g und 4 °C für 30 min ergibt 34 ml eines proteinhaltigen Überstandes (282 mg Protein), der auf eine DEAE-Sepharose CL6B (Pharmacia) (Maße: 25 x 3 cm) -Säule aufgetragen wird. Es wird mit ml Puffer (50 mM Kaliumphosphat, pH 8.0, 25 % Glycerin) gewaschen und mit 400 ml eines linearen Gradienten von 50 bis 300 mM Kaliumphosphat, pH 8.0, 25 % Glycerin eluiert. Die Fließgeschwindigkeit beträgt ca. 50 ml/h. Es werden Fraktionen von je 10 ml aufgefangen, von denen je 50 mikro l zum Aktivitätstest eingesetzt werden.

Die Aktivität aufweisende Fraktionen 46 bis 69 (ca. 205 ml) werden zusammengefaßt, auf 50 % $(NH_4)_2SO_4$ eingestellt, 16 h bei 4 °C gerührt und bei 10000 x g und 4 °C für 40 min zentrifugiert. Der Niederschlag wird in 11 ml Puffer (50 mM Kaliumphosphat, pH 8.0, 25 % Glycerin) aufgenommen und 16 h gegen 1 l desselben Puffers dialysiert (Proteinausbeute 45 mg).

Insgesamt 5 mg Protein werden in 4 Läufen zu je 300 mikro l auf einer Superose 12-Säule mittels FPLC (Pharmacia) nach Molekulargewicht aufgetrennt und bei einer Fließgeschwindigkeit von 0.3 ml/min mit 0.05 M $NH_4HCO_3$-Puffer in Fraktionen von 1 ml eluiert.

Die nadB-Enzymaktivität enthaltenden Fraktionen werden zusammengefaßt (15 ml = 2.3 mg Protein) und gefriergetrocknet. Das getrocknete Protein wird in 2 ml Puffer (50 mM $KH_2PO_4$, pH 8.0, 25 % Glycerin) aufgenommen und 16 h gegen 1 l des gleichen Puffers dialysiert.

250 mikro l (= 87 mikro g Protein) werden einer nochmaligen FPLC (wie oben) unterworfen; 2 - 5 mikro g Protein jeder proteinhaltigen Eluatfraktion werden einer SDS-PAGE mit anschließender Silberfärbung unterworfen. PAGE werden nach Lämmli (U.K. Lämmli, Nature 227 (1970) 680), Silberfärbung nach B.R. Oakley, D.R. Kirsch, N.R. Morris, Anal. Biochem. 105 (1980) 361, durchgeführt.

Das aus FPLC und PAGE bestimmte Molekulargewicht beträgt 60000 +/- 2000 Dalton.

Tabelle 1 faßt die Ergebnisse der Reinigungsschritte zusammen.

Tabelle 1

| Reinigungsschema des nadB-Enzyms | | | | |
|---|---|---|---|---|
| | Volumen (ml) | Proteinmenge (mg) | Aktivität (U) | spez. Aktivität (U/mg) |
| Rohextrakt | 220 | 4300 | 20700 | 5 |
| 50 %-$(NH_4)_2SO_4$ | 34 | 280 | 14270 | 51 |
| DEAE-Sepharose | 11 | 45 | 10780 | 240 |
| Superose-12 *) | 8 | 20 | 4300 | 215 **) |

*) experimentelle Werte umgerechnet auf Gesamtmenge

**) Der Rückgang der spezifischen Aktivität ist darauf zurückzuführen, daß die FPLC-Reinigung zum Zwecke der Probensequenzierung mit dem Ziel einer hochreinen Proteinfraktion und nicht dem Ziel maximaler Aktivität durchgeführt wurde.

c. N-terminale Aminosäuresequenz

350 bzw. 250 pmol des über FPLC gereinigten Proteins (nur eine einzige Bande auf PAGE) werden je einer vollautomatischen Sequenzanalyse durch Edmann-Abbau im Gasphasen-Sequenator 470A der Firma Applied Biosystems unterzogen.

Für den zweiten Sequenzierlauf wird die Probe mit Perameisensäure oxidiert, um die Existenz des nach dem 1. Lauf in Position 9 vermuteten Cysteins nachzuweisen. Die ermittelte N-terminale Aminosäuresequenz des nadB-Enzyms lautet:

```
Met Asn Thr Leu Pro Glu His Ser Cys Asp Val Leu Ile Ile
Gly Ser Gly Ala ...
```

Die aus der Aminosäuresequenz anhand der bekannten Codon-Zuordnung abgeleitete Nukleotidsequenz wird zur Identifizierung des Startcodons des nadB-Strukturgens verwendet und stimmt exakt mit der nach Sanger bestimmten Nukleotidsequenz (Abb. 2 b) überein.

Beispiel 6

Konstruktion des E.coli K12 Stammes RF1

Für die Identifizierung des nadA-Gens wird durch Transduktion, ausgehend von E.coli K12 C600, eine Mutante konstruiert, die

1. eine hohe Transformationseffizienz besitzt
2. rekombinante Plasmide nicht durch Rekombination mit genomischen Sequenzen verändert (recA)
3. im nadA-Genlocus mutiert ist (nadA).

Vorgehen:

Durch Transduktion des nadA50::Tn10-Genlocus aus dem Stamm NK6033 mittels Phagen T4GT7 in den Stamm C600 und Selektion auf LB/Tc-Medium wird eine Mutante E.coli K12 C600 (nadA50::Tn10) erhalten. Das für Tetracyclin-Resistenz codierende Transposon Tn10 wird nach Bochner (B.R. Bochner, H.-C. Huang, G.L. Schieven, B.N. Ames, J. Bacteriol, 143 (1980) 926-933) entfernt, und es wird eine Mutante E.coli K12 C600 (nadA) erhalten. Anschließend wird durch Transduktion mittels Phagen T4GT7 nach Beispiel 2 der recA56-Genlocus aus dem Stamm LA5708 (srl300::Tn10, recA56) in die Mutante C600 (nadA50::Tn10) eingeführt:
Selektion auf recA erfolgt auf LB-Agarplatten durch Belichten mit UV-Licht (UV-Handlampe Fluotest, Hanau, Typ 204AC; 254 nm, 0-30 s, 15 cm Abstand zwischen Lampe und Platte). Kolonien, die eine UV-Belichtung von länger als 15 s nicht überlebten, wurden als recA-Mutanten identifiziert.

Die erhaltene Mutante E.coli K12 C600 (nadA50,srl300::Tn10, recA56) wird mit RF1 bezeichnet.

Beispiel 7

Klonierung genomischer nadA-Sequenzen

75 mikro g chromosomaler DNA aus E.coli (Beispiel 1) werden mit 300 Units der Restriktionsendonuclease Sau3A bei 37 °C 2 h hydrolysiert und mit 10 mikro g des mit 10 Units BamHI hydrolysierten Plasmids pLG339 zur Ligation eingesetzt. Es erfolgt Transformation in den neu konstruierten Stamm E.coli RF1 und Selektion auf YP/Ap-Medium auf Komplementation des nadA-Mangels. Es wird ein Klon isoliert, der ein Plasmid (pCH200) mit einem ca. 12 kbp großen Insert enthält. Retransformation in die nadA-Mutante E.coli 431 bestätigt nadA-Komplementation.

Das Plasmid pCH200 wird einer partiellen Hydrolyse mit der Restriktionsendonuclease AluI unterworfen. Das Hydrolysat wird in das mit HincII hydrolysierte Plasmid pUC18 ligiert. Es folgt Transformation in den Stamm 431 und Selektion auf YP/Ap-Medium. Es wird ein Plasmid (pCH201) mit einem 1.4 kbp großen Insert isoliert (Restriktionskarte vergleiche Abb. 3). Durch Hydrolyse mit den Restriktionsendonukleasen BamHI und PstI wird das 1.4 kbp-Insert aus pCH201 gewonnen und in die BamHI/PstI-Schnittstellen des Vektors pKT235 zu dem Plasmid pCH202 inseriert.

Beispiel 8

Bestimmung der Nukleotidsequenz der nadA-Gens

Die Sequenzierung des nadA-Gens folgt der in Abb. 4 a dargestellten Strategie. Alle Fragmente werden in die entsprechenden Schnittstellen der Polylinkersequenz von pUC18 kloniert.

Abb. 4 b gibt die (doppelsträngig) bestimmte Nukleotidsequenz wieder.

Beispiel 9

Konstruktion des Plasmids pCH400

Zunächst wird das 1.4 kbp EcoRI/HindIII-Fragment aus pCH201 zwischen die EcoRI- und HindIII-Schnittstellen des Plasmids pBR322 inseriert.

Hierzu werden 1 mikro g des multi-copy Plasmids pBR322 sowie 1 mikro g des Plasmids pCH201 mit den Restriktionsendonucleasen EcoRI und HindIII quantitativ hydrolysiert; die Hydrolysate werden nach Extraktion der Enzyme mit Phenol und Fällung der DNA mit Ethanol zur Ligation mit 10 Units T4-Ligase in

20 mikro l Ligasepuffer eingesetzt und in die nadA-Mutante 431 transformiert; Selektion auf Einbau des 1.4 kbp Fragments, das nadA enthält, erfolgt auf YP/Ap-Medium.

Zwischen die HindIII- und NruI-Schnittstellen des so erhaltenen Plasmids pCH203 wird in analoger Vorgehensweise das nadB-enthaltende 3.2 kbp HindIII/NruI-Fragment aus pCH101 inseriert. Das erhaltene Plasmid pCH400 enthält beide Gene, nadA- und nadB-Gen, unter der Expressionskontrolle der genomischen Promotoren (Abb. 5).

Beispiel 10

Konstruktion des E.coli K12 Stammes RF2

Für den experimentellen Nachweis, daß ein nadA-Gen enthaltendes rekombinantes Plasmid die Chinolinsäuresynthese bewirkt, wird, ausgehend von der Chinolinsäureausscheidenden nadC-Mutante W4546, eine nadA50::Tn10, nadC-Mutante konstruiert.

Vorgehen:

Der nadA::Tn10-Genlocus aus dem Stamm NK6033 wird durch Transduktion mittels Phagen T4GT7 in den Stamm W4546 eingeführt.

Der Nachweis einer erfolgreichen Transduktion erfolgt durch Selektion auf YP/Tc-Medium unter Zusatz von Nikotinsäure ($10^{-6}$ mol/l) sowie durch HPLC-Analyse (vergleiche Beispiel 11) der Nährlösung, in der die auf YP/Tc-Medium isolierten Mutanten kultiviert worden waren. Eine nadA::Tn10-nadC-Mutante darf keine Chinolinsäure ins Medium ausscheiden. Die erhaltene Mutante E.coli K12 (nadA/nadC) wird mit RF2 bezeichnet.

Beispiel 11

Mikrobielle Erzeugung von Chinolinsäure - Erhöhung der Chinolinsäureproduktion eines Mikroorganismus durch Transformation mit Plasmiden, die nadB- und nadA-DNA-Sequenzen enthalten

Mikroorganismen des Stammes RF2 aus Beispiel 10 werden mit den Plasmiden pCH101 und pCH202 sowie mit dem Plasmid pCH400 transformiert. Die resultierenden Stämme RF2 (pCH101, pCH202) und RF2 (pCH400) sowie der Ausgangsstamm RF2 werden in YP-Medium unter Zusatz von Nicotinsäure ($10^{-6}$ mol/l) bei 37 °C für 48 h inkubiert (z. B. 5 ml Schüttelkulturen in Reagenzgläsern; z. B. 1000 ml Schüttelkultur in Erlenmeyerkolben). Unter gleichen Bedingungen wird als Referenz die Chinolinsäure-ausscheidende nadC-Mutante W4546 kultiviert. Die Ergebnisse werden mit Literaturwerten verglichen.

Weiterhin wurden alle genannten Stämme sowie ein weiterer transformierter Stamm RW1 (pCH400) in einem nährstoffreichen Medium A kultiviert.

Für die Kultivierung plasmidhaltiger Stämme in YP- bzw. A-Medium werden die benötigten Antibiotika zugesetzt, Ap für pCW400; Ap + Km für pCH101 + pCH202, pCH203 + pCH103 und pCH104 + pCH204. Für die Kultivierung des Stammes RF2 und seiner transformierten Derivate wird zusätzlich Tc zugesetzt.

Nach Bestimmung der Zelldichte durch Messung der optischen Dichte bei 550 nm werden die Zellen abzentrifugiert. Die im Überstand gelöste Chinolinsäure wird durch HPLC auf einer Anionenaustauschersäule Zorbax-$NH_2$ (Dupont) quantitativ bestimmt.

Die Ergebnisse der Messungen sind in Tabelle 2 zusammengestellt.

Tabelle 2

| Mikroorganismus | Zelldichte in YP/ Medium A (OD 550 nm) | | Konzentration Chinolinsäure in YP/ Medium A (mg/l) | |
|---|---|---|---|---|
| | YP | Medium A | YP | Medium A |
| RF2 (Medium ohne Ap) | 0.80 | 11.2 | 0 | 0 |
| RF2 (pCH101, pCH202) | 0.75 | 10.8 | 86 | 920 |
| RF2 (pCH400) | 0.89 | 9.8 | 98 | 232 |
| RW1 (pCH400) | --- | 14.0 | -- | 550 |
| W4546 (Literatur) | --- | 1.13 * | -- | 20 * |
| eigene Messung | 0.8 | 4.4 | 6 | 27 |
| W4546 (pCH203/pCH103) | 1.5 | 4.65 | 59 | 74 |
| W4546 (pCH104/pCH204) | 1.1 | 4.2 | 19 | 140 |

* J.L.R. Chandler, R.K. Gholson, J. Bacteriol. 111 (1972) 96-101

Die Stämme RF2 (pCH400) und RW1 (pCH400) sind als erfindungsgemäße Mikroorganismen bei der Deutschen Sammlung von Mikroorganismen (DSM), Braunschweig, hinterlegt.

RF2 (pCH400)

Hinterlegungsdatum : 21.01.1987
Hinterlegungsnummer: DSM 3955

RW1 (pCH400)

Hinterlegungsdatum : 14.01.1988
Hinterlegungsnummer: DSM 4362

**Patentansprüche**

1. Plasmid, das für die Fähigkeit zur Chinolinsäuresynthese codiert, **dadurch gekennzeichnet**, daß es für zwei Enzyme des NAD-Stoffwechsel codierende, genomisch getrennt kartierte Gene als DNA-Sequenzen in Verbindung mit einer, bzw. je einer Expressions-Kontroll-Sequenz enthält, und zwar
   a) die für ein Polypeptid mit der biologischen Aktivität des Enzyms Chinolinsäuresynthase codierende DNA-Sequenz nadA:

```
                 5'-CTTACT  CCTGCAAGCA  ACTCTATGTC
GGTGGAATTA  GGCGTAAAAT  GACGCATCCT  GCACATTAGG
CGTAATTCGA  GTGACTTTTC  CCCACCATTC  GACTATCTTG
TTTAGCATAT  AAAACAAATT  ACACCGATAA  CAGCGAATAT
TACGCTAATG  TCGGTTTTAA  CGTTAAGCCT  GTAAAACGAG
ATGGTAAGAT  GAGCGTAATG  TTTGATCCAG  ACACGGCGAT
TATCCTTTCC  CCCCGAAGCC  GACGCCGTTA  AGCATTGATG
AAAAAGCGTA  TTACCGCGAG  AAGATAAAAC  TGCGTCTAAA
AGAACGTAAT  GCGGTGATGG  TTGCCCACTA  CTATACCGAT
CCCGAAATTC  AACAACTGGC  AGAAGAAACC  GGTGGCTGTA
TTTCTGATTC  TCTGGAAATG  GCGCGCTTCG  GTGCAAAGCA
```

TCCCGCTTCT ACTTTGTTAG TCGCTGGGGT GAGATTTATG
GGAGAAACCG CCAAAATTCT CAGTCCGGAA AAAACAATTC
TGATGCCGAC ACTTCAGGCT GAATGTTCAC TGGATCTCGG
CTGCCCTGTT GAAGAATTTA ACGCATTTTG CGATGCCCAT
CCCGATCGTA CTGTCGTCGT CTACGCCAAC ACTTCTGCTG
CGGTAAAAGC GCGCGCAGAT TGGGGTGTAA CTTCAAGCAT
TGCCGTCGAA CTTATTGATC ATCTTGATAG TTTGTGTGAA
AAAATCATCT GGGCACCCGA CAAACATCTG GGGCGTTACG
TGCAAAAACA GACGGGTGGA GACATTCTAT GCTGGCAGGG
TGCCTGTATT GTGCATGATG AATTTAAGAC TCAGGCGTTA
ACCCGCTTGC AAGAAGAATA CCCGGATGCT GCCATACTGG
TGCATCCAGA ATCACCACAA GCTATTGTCG ATATGGCGGA
TGCGGTCGGT TCCACCAGTC AACTGATCGC TGCTGCGAAA
ACATTGCCAC ATCAGAGGCT TATTGTGGCA ACCGATCGGG

GTATTTTCTA CAAAATGCAG CAGGCGGTGC CAGATAAAGA
GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC
ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

und

b) die für ein Polypeptid mit der biologischen Aktivität des Enzyms L-Aspartatoxidase codierende DNA-Sequenz nadB:

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC
GCGGTTGATC TGATTGTTTC TGACAAAATT GGCCTGCCGG
GCACGCGACG GGTTGTTGGC GCGTGGGTAT GGAACCGTAA
TAAAGAAACG GTGGAAACCT GCCACGCAAA AGCGGTGGTG
CTGGCAACCG GCGGTGCGTC GAAGGTTTAT CAGTACACCA
CCAATCCGGA TATTTCTTCT GGCGATGGCA TTGCTATGGC
GTGGCGCGCA GGCTGCCGGG TTGCCAATCT CGAATTTAAT
CAGTTCCACC CTACCGCGCT ATATCACCCA CAGGCACGCA
ATTTCCTGTT AACAGAAGCA CTGCGCGGCG AAGGCGCTTA
TCTCAAGCGC CCGGATGGTA CGCGTTTTAT GCCCGATTTT
GATGAGCGCG GCGAACTGGC CCCGCGCCAT ATTGTCGCCC
GCGCCATTGA CCATGAAATG AAACGCCTCG GCGCAGATTG
TATGTTCCTT GATATCAGCC ATAAGCCCGC CGATTTTATT
CGCCAGCATT TCCCGATGAT TTATGAAAAG CTGCTCGGGC
TGGGGATTGA TCTCACACAA GAACCGGTAC CGATTGTGCC
TGCTGCACAT TATACCTGCG GTGGTGTAAT GGTTGATGAT
CATGGGCGTA CGGACGTCGA GGGCTTGTAT GCCATTGGCG
AGGTGAGTTA TATCGGCTTA CACGGCGCTA ACCGCATGGC
CTCGAATTCA TTGCTGGAGT GTCTGGTCTA TGGCTGGTCG

GCGGCGGAAG ATATCACCAG ACGTATGCCT TATGCCCACG
ACATCAGTAC GTTACCGCCG TGGGATGAAA GCCGCGTTGA
GAACCCTGAC GAACGGGTAG TAATTCAGCA TAACTGGCAC
GAGCTACGTC TGTTTATGTG GGATTACGTT GGCATTGTGC
GCACAACGAA GCGCCTGGAA CGCGCCCTGC GGCGGATAAC
CATGCTCCAA CAAGAAATAG ACGAATATTA CGCCCATTTC
CGCGTCTCAG ATAATTTGCT GGAGCTGCGT AATCTGGTAC

AGGTTGCCGA GTTGATTGTT CGCTGTGCAA TGATGCGTAA
AGAGAGTCGG GGGTTGCATT TCACGCTGGA TTATCCGGAA
CTGCTCACCC ATTCCGGTCC GTCGATCCTT TCCCCCGGCA
ATCATTACAT AAACAGATAA AAAGCCTGGG TCAGCGCCGT
ATAC-3'

2. Plasmid gemäß Anspruch 1, **dadurch gekennzeichnet**, daß als Expressions-Kontroll-Sequenz die genomischen Regulationssequenzen des Plasmides wirken.

3. Verwendung eines Plasmids gemäß Anspruch 1 zur Transformierung von Mikroorganismen, die anschließend unter Wachstumsbedingungen in Gegenwart eines geeigneten Nährmediums Chinolinsäure ausscheiden.

4. Verwendung der Plasmide gemäß Anspruch 1 zur Transformierung eines E. coli-Stammes.

5. Mikroorganismus, **dadurch gekennzeichnet**, daß er durch ein Plasmid gemäß Anspruch 1 transformiert ist und unter Wachstumsbedingungen in Gegenwart eines geeigneten Nährmediums Chinolinsäure ausscheidet.

6. Mikroorganismus, **dadurch gekennzeichnet**, daß er durch ein Plasmid, das eine mit einer Expressions-Kontroll-Sequenz verbundene, für ein Polypeptid mit der biologischen Aktivität des Enzyms Chinolinsäuresynthase codierende DNA-Sequenzen der folgenden Reihenfolge

5'-CTTACT CCTGCAAGCA ACTCTATGTC
GGTGGAATTA GGCGTAAAAT GACGCATCCT GCACATTAGG
CGTAATTCGA GTGACTTTTC CCCACCATTC GACTATCTTG
TTTAGCATAT AAAACAAATT ACACCGATAA CAGCGAATAT
TACGCTAATG TCGGTTTTAA CGTTAAGCCT GTAAAACGAG
ATGGTAAGAT GAGCGTAATG TTTGATCCAG ACACGGCGAT
TATCCTTTCC CCCCGAAGCC GACGCCGTTA AGCATTGATG
AAAAAGCGTA TTACCGCGAG AAGATAAAAC TGCGTCTAAA
AGAACGTAAT GCGGTGATGG TTGCCCACTA CTATACCGAT
CCCGAAATTC AACAACTGGC AGAAGAAACC GGTGGCTGTA
TTTCTGATTC TCTGGAAATG GCGCGCTTCG GTGCAAAGCA
TCCCGCTTCT ACTTTGTTAG TCGCTGGGGT GAGATTTATG
GGAGAAACCG CCAAAATTCT CAGTCCGGAA AAAACAATTC
TGATGCCGAC ACTTCAGGCT GAATGTTCAC TGGATCTCGG
CTGCCCTGTT GAAGAATTTA ACGCATTTTG CGATGCCCAT
CCCGATCGTA CTGTCGTCGT CTACGCCAAC ACTTCTGCTG
CGGTAAAAGC GCGCGCAGAT TGGGGTGTAA CTTCAAGCAT
TGCCGTCGAA CTTATTGATC ATCTTGATAG TTTGTGTGAA
AAAATCATCT GGGCACCCGA CAAACATCTG GGGCGTTACG
TGCAAAAACA GACGGGTGGA GACATTCTAT GCTGGCAGGG
TGCCTGTATT GTGCATGATG AATTTAAGAC TCAGGCGTTA
ACCCGCTTGC AAGAAGAATA CCCGGATGCT GCCATACTGG
TGCATCCAGA ATCACCACAA GCTATTGTCG ATATGGCGGA
TGCGGTCGGT TCCACCAGTC AACTGATCGC TGCTGCGAAA
ACATTGCCAC ATCAGAGGCT TATTGTGGCA ACCGATCGGG

GTATTTTCTA CAAAATGCAG CAGGCGGTGC CAGATAAAGA

GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC
ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

und gleichzeitig durch ein Plasmid, das eine mit einer Expressions-Kontroll-Sequenz verbundene, für ein Polypeptid mit der biologischen Aktivität des Enzyms L-Aspartatoxidase codierende DNA-Sequenzen der folgenden Reihenfolge

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC
GCGGTTGATC TGATTGTTTC TGACAAAATT GGCCTGCCGG

GCACGCGACG GGTTGTTGGC GCGTGGGTAT GGAACCGTAA
TAAAGAAACG GTGGAAACCT GCCACGCAAA AGCGGTGGTG
CTGGCAACCG GCGGTGCGTC GAAGGTTTAT CAGTACACCA
CCAATCCGGA TATTTCTTCT GGCGATGGCA TTGCTATGGC
GTGGCGCGCA GGCTGCCGGG TTGCCAATCT CGAATTTAAT
CAGTTCCACC CTACCGCGCT ATATCACCCA CAGGCACGCA
ATTTCCTGTT AACAGAAGCA CTGCGCGGCG AAGGCGCTTA
TCTCAAGCGC CCGGATGGTA CGCGTTTTAT GCCCGATTTT
GATGAGCGCG GCGAACTGGC CCCGCGCCAT ATTGTCGCCC
GCGCCATTGA CCATGAAATG AAACGCCTCG GCGCAGATTG
TATGTTCCTT GATATCAGCC ATAAGCCCGC CGATTTTATT
CGCCAGCATT TCCCGATGAT TTATGAAAAG CTGCTCGGGC
TGGGGATTGA TCTCACACAA GAACCGGTAC CGATTGTGCC
TGCTGCACAT TATACCTGCG GTGGTGTAAT GGTTGATGAT
CATGGGCGTA CGGACGTCGA GGGCTTGTAT GCCATTGGCG
AGGTGAGTTA TATCGGCTTA CACGGCGCTA ACCGCATGGC
CTCGAATTCA TTGCTGGAGT GTCTGGTCTA TGGCTGGTCG
GCGGCGGAAG ATATCACCAG ACGTATGCCT TATGCCCACG
ACATCAGTAC GTTACCGCCG TGGGATGAAA GCCGCGTTGA
GAACCCTGAC GAACGGGTAG TAATTCAGCA TAACTGGCAC
GAGCTACGTC TGTTTATGTG GGATTACGTT GGCATTGTGC
GCACAACGAA GCGCCTGGAA CGCGCCCTGC GGCGGATAAC
CATGCTCCAA CAAGAAATAG ACGAATATTA CGCCCATTTC
CGCGTCTCAG ATAATTTGCT GGAGCTGCGT AATCTGGTAC

AGGTTGCCGA GTTGATTGTT CGCTGTGCAA TGATGCGTAA
AGAGAGTCGG GGGTTGCATT TCACGCTGGA TTATCCGGAA
CTGCTCACCC ATTCCGGTCC GTCGATCCTT TCCCCCGGCA
ATCATTACAT AAACAGATAA AAAGCCTGGG TCAGCGCCGT
ATAC-3'

transformiert ist und unter Wachstumsbedingunen in Gegenwart eines geeigneten Nährmediums Chinolinsäure ausscheidet.

## Claims

1. A plasmid which codes for the ability to synthesize quinolinic acid, characterized in that in contains genomically separately mapped genes as DNA sequnces which code for two enzymes of the NAD metabolism and which are combined with one respective each with one expression control sequence, the DNA sequences are

a. coding for a polypeptide with the biological activity of the enzyme quinolinic acid synthase the sequenze nadA:

| | | | |
|---|---|---|---|
| | 5'-CTTACT | CCTGCAAGCA | ACTCTATGTC |
| GGTGGAATTA | GGCGTAAAAT | GACGCATCCT | GCACATTAGG |
| CGTAATTCGA | GTGACTTTTC | CCCACCATTC | GACTATCTTG |
| TTTAGCATAT | AAAACAAATT | ACACCGATAA | CAGCGAATAT |
| TACGCTAATG | TCGGTTTTAA | CGTTAAGCCT | GTAAAACGAG |
| ATGGTAAGAT | GAGCGTAATG | TTTGATCCAG | ACACGGCGAT |
| TATCCTTTCC | CCCCGAAGCC | GACGCCGTTA | AGCATTGATG |
| AAAAAGCGTA | TTACCGCGAG | AAGATAAAAC | TGCGTCTAAA |
| AGAACGTAAT | GCGGTGATGG | TTGCCCACTA | CTATACCGAT |
| CCCGAAATTC | AACAACTGGC | AGAAGAAACC | GGTGGCTGTA |

| | | | |
|---|---|---|---|
| TTTCTGATTC | TCTGGAAATG | GCGCGCTTCG | GTGCAAAGCA |
| TCCCGCTTCT | ACTTTGTTAG | TCGCTGGGGT | GAGATTTATG |
| GGAGAAACCG | CCAAAATTCT | CAGTCCGGAA | AAAACAATTC |
| TGATGCCGAC | ACTTCAGGCT | GAATGTTCAC | TGGATCTCGG |
| CTGCCCTGTT | GAAGAATTTA | ACGCATTTTG | CGATGCCCAT |
| CCCGATCGTA | CTGTCGTCGT | CTACGCCAAC | ACTTCTGCTG |
| CGGTAAAAGC | GCGCGCAGAT | TGGGGTGTAA | CTTCAAGCAT |
| TGCCGTCGAA | CTTATTGATC | ATCTTGATAG | TTTGTGTGAA |
| AAAATCATCT | GGGCACCCGA | CAAACATCTG | GGGCGTTACG |
| TGCAAAAACA | GACGGGTGGA | GACATTCTAT | GCTGGCAGGG |
| TGCCTGTATT | GTGCATGATG | AATTTAAGAC | TCAGGCGTTA |
| ACCCGCTTGC | AAGAAGAATA | CCCGGATGCT | GCCATACTGG |
| TGCATCCAGA | ATCACCACAA | GCTATTGTCG | ATATGGCGGA |
| TGCGGTCGGT | TCCACCAGTC | AACTGATCGC | TGCTGCGAAA |
| ACATTGCCAC | ATCAGAGGCT | TATTGTGGCA | ACCGATCGGG |
| GTATTTTCTA | CAAAATGCAG | CAGGCGGTGC | CAGATAAAGA |
| GTTACTGGAA | GCACCAACCG | CAGGTGAGGG | TGCAACCTGC |
| CGCAGCTGCG | CGCATTGTCC | GTGGATGGCC | ATGAATGCCT |
| TAGGCCATCG | CAGAGGCATT | AGAACAGGAA | GGAAGCAATC |
| ACGAGGTTCA | TGTTGATGAA | AGGCTGCGGA | GAAGGGCGCT |
| GGTGCCGCTC | AATCGTATGC | TGGATTTTGC | GGCTACACTA |
| CGTGGATAAC | GAATAATAAG | GCGTAACGTT | ACGCTTTGGG |
| GGAAAGATGG | ATTTTTTTAG | TGTGCAGAAT | ATCCTGGTAC |
| ATATACCAAT | AGGGGCAGGC | GGTTATGATC | TCTCATGGAT |
| CGAAGCGGTA | GGCACGATCG | CCGGGTTGCT | GTGTATTGGC |
| CTTGCCAGTC | TGGAGAAGAT | CAGCAACTAC | TTCTTTGGCC |
| TGATCAACGT | CACCTTGTTT | GGCATTATTT | TCTTTCAGAT |
| TCAG-3' | | | |

and

b) coding for a polypeptide with the bioligical acitvity of the enzyme L-aspartate oxidase the sequence nadB:

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC
GCGGTTGATC TGATTGTTTC TGACAAAATT GGCCTGCCGG
GCACGCGACG GGTTGTTGGC GCGTGGGTAT GGAACCGTAA
TAAAGAAACG GTGGAAACCT GCCACGCAAA AGCGGTGGTG
CTGGCAACCG GCGGTGCGTC GAAGGTTTAT CAGTACACCA
CCAATCCGGA TATTTCTTCT GGCGATGGCA TTGCTATGGC
GTGGCGCGCA GGCTGCCGGG TTGCCAATCT CGAATTTAAT
CAGTTCCACC CTACCGCGCT ATATCACCCA CAGGCACGCA
ATTTCCTGTT AACAGAAGCA CTGCGCGGCG AAGGCGCTTA
TCTCAAGCGC CCGGATGGTA CGCGTTTTAT GCCCGATTTT
GATGAGCGCG GCGAACTGGC CCCGCGCCAT ATTGTCGCCC
GCGCCATTGA CCATGAAATG AAACGCCTCG GCGCAGATTG
TATGTTCCTT GATATCAGCC ATAAGCCCGC CGATTTTATT
CGCCAGCATT TCCCGATGAT TTATGAAAAG CTGCTCGGGC
TGGGGATTGA TCTCACACAA GAACCGGTAC CGATTGTGCC
TGCTGCACAT TATACCTGCG GTGGTGTAAT GGTTGATGAT

CATGGGCGTA CGGACGTCGA GGGCTTGTAT GCCATTGGCG
AGGTGAGTTA TATCGGCTTA CACGGCGCTA ACCGCATGGC
CTCGAATTCA TTGCTGGAGT GTCTGGTCTA TGGCTGGTCG
GCGGCGGAAG ATATCACCAG ACGTATGCCT TATGCCCACG
ACATCAGTAC GTTACCGCCG TGGGATGAAA GCCGCGTTGA
GAACCCTGAC GAACGGGTAG TAATTCAGCA TAACTGGCAC
GAGCTACGTC TGTTTATGTG GGATTACGTT GGCATTGTGC
GCACAACGAA GCGCCTGGAA CGCGCCCTGC GGCGGATAAC
CATGCTCCAA CAAGAAATAG ACGAATATTA CGCCCATTTC
CGCGTCTCAG ATAATTTGCT GGAGCTGCGT AATCTGGTAC

AGGTTGCCGA GTTGATTGTT CGCTGTGCAA TGATGCGTAA
AGAGAGTCGG GGGTTGCATT TCACGCTGGA TTATCCGGAA
CTGCTCACCC ATTCCGGTCC GTCGATCCTT TCCCCCGGCA
ATCATTACAT AAACAGATAA AAAGCCTGGG TCAGCGCCGT
ATAC-3'

2. Plasmid of claim 1 characterized in that the genomically regulation sequences of the plasmid act as expression control sequenz.

3. Use of a plasmid of claim 1 for the transformation of microorganisms which eliminate quinolicic acid when helt under growth conditions in a suitable nutrient medium.

4. Use of the plasmids of claim 1 for the transformation of a strain of E. coli.

5. Microorganism, characterized in that it is transformed by a plasmid of claim 1 and which eliminates quinolinic acid under growth conditions in a suitable nutrient medium.

6. Microorganism, characterized in that it is transformed with a plasmid which is combined with an express control sequenz coding for a polypeptide with the biological activity of the enzyme quinolinic acid synthase having the DNA sequences:

```
                      5'-CTTACT  CCTGCAAGCA  ACTCTATGTC
GGTGGAATTA  GGCGTAAAAT  GACGCATCCT  GCACATTAGG
CGTAATTCGA  GTGACTTTTC  CCCACCATTC  GACTATCTTG
TTTAGCATAT  AAAACAAATT  ACACCGATAA  CAGCGAATAT
TACGCTAATG  TCGGTTTTAA  CGTTAAGCCT  GTAAAACGAG
ATGGTAAGAT  GAGCGTAATG  TTTGATCCAG  ACACGGCGAT
TATCCTTTCC  CCCCGAAGCC  GACGCCGTTA  AGCATTGATG
AAAAAGCGTA  TTACCGCGAG  AAGATAAAAC  TGCGTCTAAA
AGAACGTAAT  GCGGTGATGG  TTGCCCACTA  CTATACCGAT
CCCGAAATTC  AACAACTGGC  AGAAGAAACC  GGTGGCTGTA
TTTCTGATTC  TCTGGAAATG  GCGCGCTTCG  GTGCAAAGCA
TCCCGCTTCT  ACTTTGTTAG  TCGCTGGGGT  GAGATTTATG
GGAGAAACCG  CCAAAATTCT  CAGTCCGGAA  AAAACAATTC
TGATGCCGAC  ACTTCAGGCT  GAATGTTCAC  TGGATCTCGG
CTGCCCTGTT  GAAGAATTTA  ACGCATTTTG  CGATGCCCAT
CCCGATCGTA  CTGTCGTCGT  CTACGCCAAC  ACTTCTGCTG
CGGTAAAAGC  GCGCGCAGAT  TGGGGTGTAA  CTTCAAGCAT
TGCCGTCGAA  CTTATTGATC  ATCTTGATAG  TTTGTGTGAA
AAAATCATCT  GGGCACCCGA  CAAACATCTG  GGGCGTTACG
TGCAAAAACA  GACGGGTGGA  GACATTCTAT  GCTGGCAGGG
TGCCTGTATT  GTGCATGATG  AATTTAAGAC  TCAGGCGTTA
ACCCGCTTGC  AAGAAGAATA  CCCGGATGCT  GCCATACTGG
TGCATCCAGA  ATCACCACAA  GCTATTGTCG  ATATGGCGGA
TGCGGTCGGT  TCCACCAGTC  AACTGATCGC  TGCTGCGAAA
ACATTGCCAC  ATCAGAGGCT  TATTGTGGCA  ACCGATCGGG
```

GTATTTTCTA CAAAATGCAG CAGGCGGTGC CAGATAAAGA
GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC
ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

and parallel with a plasmid which is combined with an express control sequenz coding for a polypeptide with the biological activity of the enzyme L-aspartate oxidase having the DNA sequences:

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC

```
GCGGTTGATC  TGATTGTTTC  TGACAAAATT  GGCCTGCCGG
GCACGCGACG  GGTTGTTGGC  GCGTGGGTAT  GGAACCGTAA
TAAAGAAACG  GTGGAAACCT  GCCACGCAAA  AGCGGTGGTG
CTGGCAACCG  GCGGTGCGTC  GAAGGTTTAT  CAGTACACCA
CCAATCCGGA  TATTTCTTCT  GGCGATGGCA  TTGCTATGGC
GTGGCGCGCA  GGCTGCCGGG  TTGCCAATCT  CGAATTTAAT
CAGTTCCACC  CTACCGCGCT  ATATCACCCA  CAGGCACGCA
ATTTCCTGTT  AACAGAAGCA  CTGCGCGGCG  AAGGCGCTTA
TCTCAAGCGC  CCGGATGGTA  CGCGTTTTAT  GCCCGATTTT
GATGAGCGCG  GCGAACTGGC  CCCGCGCCAT  ATTGTCGCCC
GCGCCATTGA  CCATGAAATG  AAACGCCTCG  GCGCAGATTG
TATGTTCCTT  GATATCAGCC  ATAAGCCCGC  CGATTTTATT
CGCCAGCATT  TCCCGATGAT  TTATGAAAAG  CTGCTCGGGC
TGGGGATTGA  TCTCACACAA  GAACCGGTAC  CGATTGTGCC
TGCTGCACAT  TATACCTGCG  GTGGTGTAAT  GGTTGATGAT
CATGGGCGTA  CGGACGTCGA  GGGCTTGTAT  GCCATTGGCG
AGGTGAGTTA  TATCGGCTTA  CACGGCGCTA  ACCGCATGGC
CTCGAATTCA  TTGCTGGAGT  GTCTGGTCTA  TGGCTGGTCG
GCGGCGGAAG  ATATCACCAG  ACGTATGCCT  TATGCCCACG
ACATCAGTAC  GTTACCGCCG  TGGGATGAAA  GCCGCGTTGA
GAACCCTGAC  GAACGGGTAG  TAATTCAGCA  TAACTGGCAC
GAGCTACGTC  TGTTTATGTG  GGATTACGTT  GGCATTGTGC
GCACAACGAA  GCGCCTGGAA  CGCGCCCTGC  GGCGGATAAC
CATGCTCCAA  CAAGAAATAG  ACGAATATTA  CGCCCATTTC
CGCGTCTCAG  ATAATTTGCT  GGAGCTGCGT  AATCTGGTAC

AGGTTGCCGA  GTTGATTGTT  CGCTGTGCAA  TGATGCGTAA
AGAGAGTCGG  GGGTTGCATT  TCACGCTGGA  TTATCCGGAA
CTGCTCACCC  ATTCCGGTCC  GTCGATCCTT  TCCCCCGGCA
ATCATTACAT  AAACAGATAA  AAAGCCTGGG  TCAGCGCCGT
ATAC-3'
```

the microorganism eliminating quinolinic acid under growth conditions in a nutrient medium.

## Revendications

1. Plasmide qui code pour la capacité de synthèse de l'acide quinolinique, caractérisé en ce qu'il contient deux séquences de DNA, qui sont des gênes génoméniquement séparés comme des séquences de DNA en connection avec une respectivement chaqun avec une séquence de contrôle de l'expression, qui codent pour deux enzymes du métabolisme du NAD qui sont

a. la DNA séquence nadA qui code pour un polypeptide avec l'activite biologique d'enzyme acide quinolinique-synthase:

```
                5'-CTTACT  CCTGCAAGCA  ACTCTATGTC
GGTGGAATTA  GGCGTAAAAT  GACGCATCCT  GCACATTAGG
CGTAATTCGA  GTGACTTTTC  CCCACCATTC  GACTATCTTG
TTTAGCATAT  AAAACAAATT  ACACCGATAA  CAGCGAATAT
TACGCTAATG  TCGGTTTTAA  CGTTAAGCCT  GTAAAACGAG
ATGGTAAGAT  GAGCGTAATG  TTTGATCCAG  ACACGGCGAT
TATCCTTTCC  CCCCGAAGCC  GACGCCGTTA  AGCATTGATG
AAAAAGCGTA  TTACCGCGAG  AAGATAAAAC  TGCGTCTAAA
AGAACGTAAT  GCGGTGATGG  TTGCCCACTA  CTATACCGAT
```

CCCGAAATTC AACAACTGGC AGAAGAAACC GGTGGCTGTA
TTTCTGATTC TCTGGAAATG GCGCGCTTCG GTGCAAAGCA
TCCCGCTTCT ACTTTGTTAG TCGCTGGGGT GAGATTTATG
GGAGAAACCG CCAAAATTCT CAGTCCGGAA AAAACAATTC
TGATGCCGAC ACTTCAGGCT GAATGTTCAC TGGATCTCGG
CTGCCCTGTT GAAGAATTTA ACGCATTTTG CGATGCCCAT
CCCGATCGTA CTGTCGTCGT CTACGCCAAC ACTTCTGCTG
CGGTAAAAGC GCGCGCAGAT TGGGGTGTAA CTTCAAGCAT
TGCCGTCGAA CTTATTGATC ATCTTGATAG TTTGTGTGAA
AAAATCATCT GGGCACCCGA CAAACATCTG GGGCGTTACG
TGCAAAAACA GACGGGTGGA GACATTCTAT GCTGGCAGGG
TGCCTGTATT GTGCATGATG AATTTAAGAC TCAGGCGTTA
ACCCGCTTGC AAGAAGAATA CCCGGATGCT GCCATACTGG
TGCATCCAGA ATCACCACAA GCTATTGTCG ATATGGCGGA
TGCGGTCGGT TCCACCAGTC AACTGATCGC TGCTGCGAAA
ACATTGCCAC ATCAGAGGCT TATTGTGGCA ACCGATCGGG

GTATTTTCTA CAAAATGCAG CAGGCGGTGC CAGATAAAGA
GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC
ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

et

b. la DNA séquence nadB qui code pour un polypeptide avec l'activité biologique d'enzyme L-aspartatoxydase:

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC
GCGGTTGATC TGATTGTTTC TGACAAAATT GGCCTGCCGG
GCACGCGACG GGTTGTTGGC GCGTGGGTAT GGAACCGTAA
TAAAGAAACG GTGGAAACCT GCCACGCAAA AGCGGTGGTG
CTGGCAACCG GCGGTGCGTC GAAGGTTTAT CAGTACACCA
CCAATCCGGA TATTTCTTCT GGCGATGGCA TTGCTATGGC
GTGGCGCGCA GGCTGCCGGG TTGCCAATCT CGAATTTAAT
CAGTTCCACC CTACCGCGCT ATATCACCCA CAGGCACGCA
ATTTCCTGTT AACAGAAGCA CTGCGCGGCG AAGGCGCTTA
TCTCAAGCGC CCGGATGGTA CGCGTTTTAT GCCCGATTTT
GATGAGCGCG GCGAACTGGC CCCGCGCCAT ATTGTCGCCC
GCGCCATTGA CCATGAAATG AAACGCCTCG GCGCAGATTG
TATGTTCCTT GATATCAGCC ATAAGCCCGC CGATTTTATT
CGCCAGCATT TCCCGATGAT TTATGAAAAG CTGCTCGGGC
TGGGGATTGA TCTCACACAA GAACCGGTAC CGATTGTGCC
TGCTGCACAT TATACCTGCG GTGGTGTAAT GGTTGATGAT

```
CATGGGCGTA  CGGACGTCGA  GGGCTTGTAT  GCCATTGGCG
AGGTGAGTTA  TATCGGCTTA  CACGGCGCTA  ACCGCATGGC
CTCGAATTCA  TTGCTGGAGT  GTCTGGTCTA  TGGCTGGTCG
GCGGCGGAAG  ATATCACCAG  ACGTATGCCT  TATGCCCACG
ACATCAGTAC  GTTACCGCCG  TGGGATGAAA  GCCGCGTTGA
GAACCCTGAC  GAACGGGTAG  TAATTCAGCA  TAACTGGCAC
GAGCTACGTC  TGTTTATGTG  GGATTACGTT  GGCATTGTGC
GCACAACGAA  GCGCCTGGAA  CGCGCCCTGC  GGCGGATAAC
CATGCTCCAA  CAAGAAATAG  ACGAATATTA  CGCCCATTTC
CGCGTCTCAG  ATAATTTGCT  GGAGCTGCGT  AATCTGGTAC

AGGTTGCCGA  GTTGATTGTT  CGCTGTGCAA  TGATGCGTAA
AGAGAGTCGG  GGGTTGCATT  TCACGCTGGA  TTATCCGGAA
CTGCTCACCC  ATTCCGGTCC  GTCGATCCTT  TCCCCCGGCA
ATCATTACAT  AAACAGATAA  AAAGCCTGGG  TCAGCGCCGT
ATAC-3'
```

2. Plasmide selon la revendication 1, caractérisé en ce que les séquences de regulation génonomiques du plasmide exercent comme des séquences de contrôl de l'expression.

3. Usage d'un plasmide selon la revendication 1, pour la transformation de micro-organismes qui en suivant séparent d'acide quinolinique sous des conditions de développement en présence d'un milieu nutritif propre.

4. Usage des plasmides selon la revendication 1, pour la transformation du souche de E. coli.

5. Micro-organisme, caractérisé en ce qu'il est transormé par un plasmide selon la revendication 1 et qui sépare d'acide quinolinique sous des conditions de développement en présence d'un milieu nutritif propre.

6. Micro-organisme, caractérisé en ce qu'il est transformé par un plasmide qui contient une DNA séquence qui est en connection avec une séquence de contrôl de l'expression et qui code pour un polypeptide avec l'acitivité biologique d'enzyme acid quinolinique-synthase avec la structure suivante:

5'-CTTACT CCTGCAAGCA ACTCTATGTC
GGTGGAATTA GGCGTAAAAT GACGCATCCT GCACATTAGG
CGTAATTCGA GTGACTTTTC CCCACCATTC GACTATCTTG
TTTAGCATAT AAAACAAATT ACACCGATAA CAGCGAATAT
TACGCTAATG TCGGTTTTAA CGTTAAGCCT GTAAAACGAG
ATGGTAAGAT GAGCGTAATG TTTGATCCAG ACACGGCGAT
TATCCTTTCC CCCCGAAGCC GACGCCGTTA AGCATTGATG
AAAAAGCGTA TTACCGCGAG AAGATAAAAC TGCGTCTAAA
AGAACGTAAT GCGGTGATGG TTGCCCACTA CTATACCGAT
CCCGAAATTC AACAACTGGC AGAAGAAACC GGTGGCTGTA
TTTCTGATTC TCTGGAAATG GCGCGCTTCG GTGCAAAGCA
TCCCGCTTCT ACTTTGTTAG TCGCTGGGGT GAGATTTATG
GGAGAAACCG CCAAAATTCT CAGTCCGGAA AAAACAATTC
TGATGCCGAC ACTTCAGGCT GAATGTTCAC TGGATCTCGG
CTGCCCTGTT GAAGAATTTA ACGCATTTTG CGATGCCCAT
CCCGATCGTA CTGTCGTCGT CTACGCCAAC ACTTCTGCTG
CGGTAAAAGC GCGCGCAGAT TGGGGTGTAA CTTCAAGCAT
TGCCGTCGAA CTTATTGATC ATCTTGATAG TTTGTGTGAA
AAAATCATCT GGGCACCCGA CAAACATCTG GGGCGTTACG
TGCAAAAACA GACGGGTGGA GACATTCTAT GCTGGCAGGG
TGCCTGTATT GTGCATGATG AATTTAAGAC TCAGGCGTTA
ACCCGCTTGC AAGAAGAATA CCCGGATGCT GCCATACTGG

TGCATCCAGA ATCACCACAA GCTATTGTCG ATATGGCGGA
TGCGGTCGGT TCCACCAGTC AACTGATCGC TGCTGCGAAA
ACATTGCCAC ATCAGAGGCT TATTGTGGCA ACCGATCGGG

GTATTTTCTA CAAAATGCAG CAGGCGGTGC CAGATAAAGA
GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC
ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

et parallelement par un plasmide qui contient une DNA séquence qui est en connection avec une séquence de contrôl de l'expression et qui code pour un polypeptide avec l'activité biologique d'encyme L-aspartatoxydase avec la structure suivante:

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT

| | | | |
|---|---|---|---|
| GCAGTTGAAT | TTGTCGCCAG | CAATGCACGA | TCCTGTGTGC |
| AATGGCTAAT | CGACCAGGGG | GTGTTGTTTG | ATACCCACAT |
| TCAACCGAAT | GGCGAAGAAA | GTTACCATCT | GACCCGTGAA |
| GGTGGACATA | GTCACCGTCG | TATTCTTCAT | GCCGCCGACG |
| CCACCGGTAG | AGAAGTAGAA | ACCACGCTGG | TGAGCAAGGC |
| GCTGAACCAT | CCGAATATTC | GCGTGCTGGA | GCGCACGAAC |
| GCGGTTGATC | TGATTGTTTC | TGACAAAATT | GGCCTGCCGG |
| GCACGCGACG | GGTTGTTGGC | GCGTGGGTAT | GGAACCGTAA |
| TAAAGAAACG | GTGGAAACCT | GCCACGCAAA | AGCGGTGGTG |
| CTGGCAACCG | GCGGTGCGTC | GAAGGTTTAT | CAGTACACCA |
| CCAATCCGGA | TATTTCTTCT | GGCGATGGCA | TTGCTATGGC |
| GTGGCGCGCA | GGCTGCCGGG | TTGCCAATCT | CGAATTTAAT |
| CAGTTCCACC | CTACCGCGCT | ATATCACCCA | CAGGCACGCA |
| ATTTCCTGTT | AACAGAAGCA | CTGCGCGGCG | AAGGCGCTTA |
| TCTCAAGCGC | CCGGATGGTA | CGCGTTTTAT | GCCCGATTTT |
| GATGAGCGCG | GCGAACTGGC | CCCGCGCCAT | ATTGTCGCCC |
| GCGCCATTGA | CCATGAAATG | AAACGCCTCG | GCGCAGATTG |
| TATGTTCCTT | GATATCAGCC | ATAAGCCCGC | CGATTTTATT |
| CGCCAGCATT | TCCCGATGAT | TTATGAAAAG | CTGCTCGGGC |
| TGGGGATTGA | TCTCACACAA | GAACCGGTAC | CGATTGTGCC |
| TGCTGCACAT | TATACCTGCG | GTGGTGTAAT | GGTTGATGAT |
| CATGGGCGTA | CGGACGTCGA | GGGCTTGTAT | GCCATTGGCG |
| AGGTGAGTTA | TATCGGCTTA | CACGGCGCTA | ACCGCATGGC |
| CTCGAATTCA | TTGCTGGAGT | GTCTGGTCTA | TGGCTGGTCG |
| GCGGCGGAAG | ATATCACCAG | ACGTATGCCT | TATGCCCACG |
| ACATCAGTAC | GTTACCGCCG | TGGGATGAAA | GCCGCGTTGA |
| GAACCCTGAC | GAACGGGTAG | TAATTCAGCA | TAACTGGCAC |
| GAGCTACGTC | TGTTTATGTG | GGATTACGTT | GGCATTGTGC |
| GCACAACGAA | GCGCCTGGAA | CGCGCCCTGC | GGCGGATAAC |
| CATGCTCCAA | CAAGAAATAG | ACGAATATTA | CGCCCATTTC |
| CGCGTCTCAG | ATAATTTGCT | GGAGCTGCGT | AATCTGGTAC |
| AGGTTGCCGA | GTTGATTGTT | CGCTGTGCAA | TGATGCGTAA |
| AGAGAGTCGG | GGGTTGCATT | TCACGCTGGA | TTATCCGGAA |

CTGCTCACCC ATTCCGGTCC GTCGATCCTT TCCCCCGGCA
ATCATTACAT AAACAGATAA AAAGCCTGGG TCAGCGCCGT
ATAC-3'

le micro-organisme séparent d'acide quinolinique sous des conditions de développement en présence d'un milieu nutritif propre.

Abbildung 1

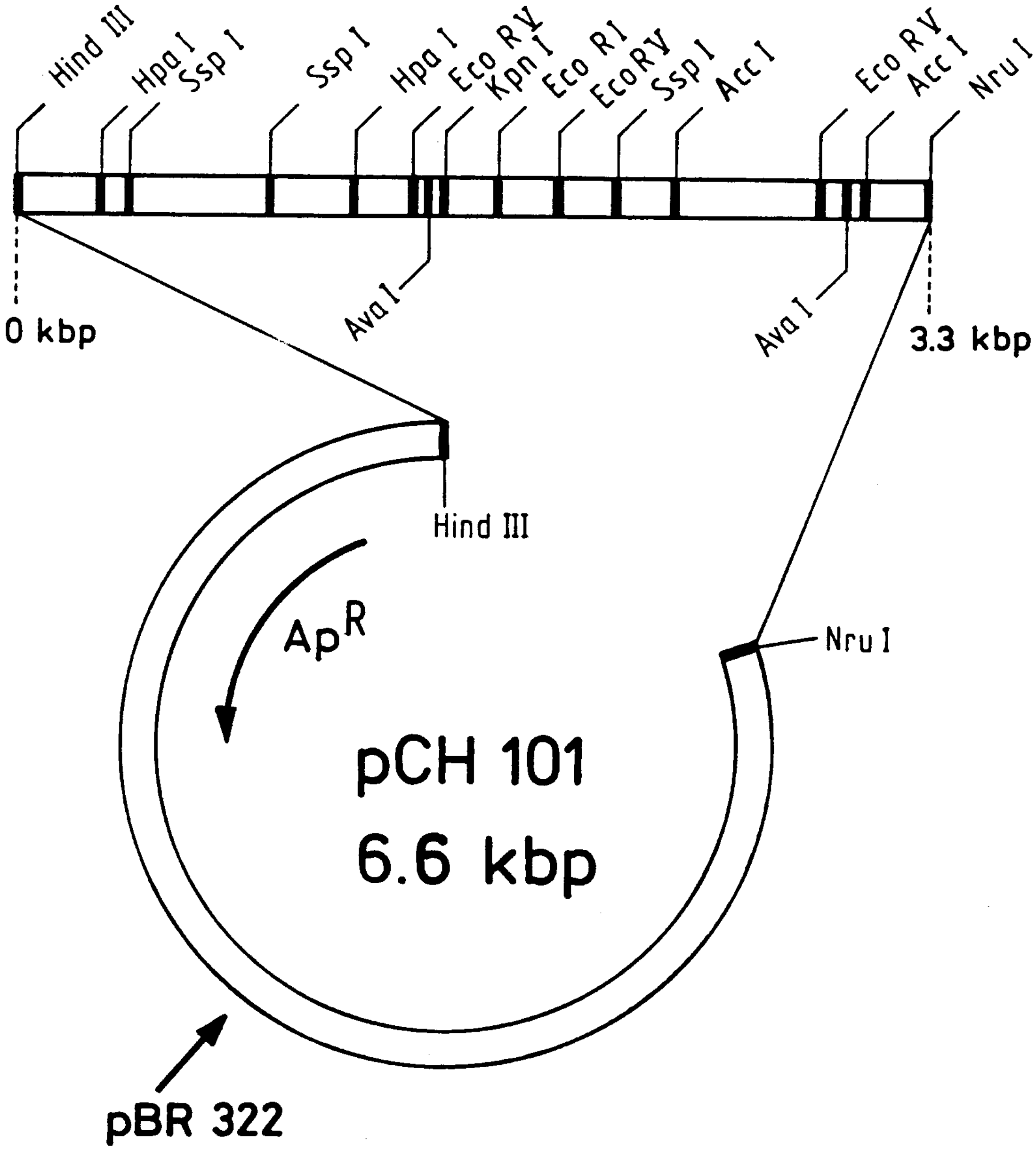


Pat. 1 - 1

Abbildung 2 a

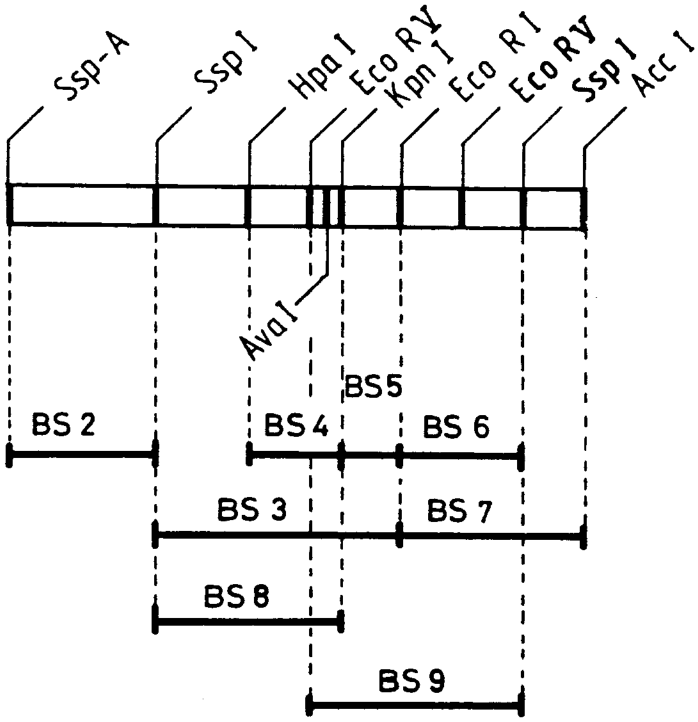


BSx = Subklone nadB-Gen in pUC 19

Pat. 1-2a

Abbildung 2 b

SspI-A

5'-ATTAAAATAG CAGGTGTTTA TCCGCACAAC ATGATGCTAT
GCTGACCAAA CCATGTTTAG TAAATTAAAC AAAGAAAATG
AATACTCTCC CTGAACATTC ATGTGACGTG TTGATTATCG
GTAGCGGCGC AGCCGGACTT TCACTGGCGC TACGCCTGGC
TGACCAGCAT CAGGTCATCG TTCTAAGTAA AGGCCCGGTA
ACGGAAGGTT CAACATTTTA TGCCCAGGGC GGTATTGCCG
CCGTGTTTGA TGAAACTGAC AGCATTGACT CGCATGTGGA
AGACACATTG ATTGCCGGGG CTGGTATTTG CGATCGCCAT
GCAGTTGAAT TTGTCGCCAG CAATGCACGA TCCTGTGTGC
AATGGCTAAT CGACCAGGGG GTGTTGTTTG ATACCCACAT
TCAACCGAAT GGCGAAGAAA GTTACCATCT GACCCGTGAA
GGTGGACATA GTCACCGTCG TATTCTTCAT GCCGCCGACG
CCACCGGTAG AGAAGTAGAA ACCACGCTGG TGAGCAAGGC
GCTGAACCAT CCGAATATTC GCGTGCTGGA GCGCACGAAC
GCGGTTGATC TGATTGTTTC TGACAAAATT GGCCTGCCGG
GCACGCGACG GGTTGTTGGC GCGTGGGTAT GGAACCGTAA
TAAAGAAACG GTGGAAACCT GCCACGCAAA AGCGGTGGTG
CTGGCAACCG GCGGTGCGTC GAAGGTTTAT CAGTACACCA
CCAATCCGGA TATTTCTTCT GGCGATGGCA TTGCTATGGC
GTGGCGCGCA GGCTGCCGGG TTGCCAATCT CGAATTTAAT
CAGTTCCACC CTACCGCGCT ATATCACCCA CAGGCACGCA
ATTTCCTGTT AACAGAAGCA CTGCGCGGCG AAGGCGCTTA
TCTCAAGCGC CCGGATGGTA CGCGTTTTAT GCCCGATTTT
GATGAGCGCG GCGAACTGGC CCCGCGCCAT ATTGTCGCCC
GCGCCATTGA CCATGAAATG AAACGCCTCG GCGCAGATTG
TATGTTCCTT GATATCAGCC ATAAGCCCGC CGATTTTATT
CGCCAGCATT TCCCGATGAT TTATGAAAAG CTGCTCGGGC
TGGGGATTGA TCTCACACAA GAACCGGTAC CGATTGTGCC

Abbildung 2 b, Fortsetzung

TGCTGCACAT TATACCTGCG GTGGTGTAAT GGTTGATGAT
CATGGGCGTA CGGACGTCGA GGGCTTGTAT GCCATTGGCG
AGGTGAGTTA TATCGGCTTA CACGGCGCTA ACCGCATGGC
CTCGAATTCA TTGCTGGAGT GTCTGGTCTA TGGCTGGTCG
GCGGCGGAAG ATATCACCAG ACGTATGCCT TATGCCCACG
ACATCAGTAC GTTACCGCCG TGGGATGAAA GCCGCGTTGA
GAACCCTGAC GAACGGGTAG TAATTCAGCA TAACTGGCAC
GAGCTACGTC TGTTTATGTG GGATTACGTT GGCATTGTGC
GCACAACGAA GCGCCTGGAA CGCGCCCTGC GGCGGATAAC
CATGCTCCAA CAAGAAATAG ACGAATATTA CGCCCATTTC
CGCGTCTCAG ATAATTTGCT GGAGCTGCGT AATCTGGTAC
AGGTTGCCGA GTTGATTGTT CGCTGTGCAA TGATGCGTAA
AGAGAGTCGG GGGTTGCATT TCACGCTGGA TTATCCGGAA
CTGCTCACCC ATTCCGGTCC GTCGATCCTT TCCCCCGGCA
ATCATTACAT AAACAGATAA AAAGCCTGGG TCAGCGCCGT
ATAC-3'

Abbildung 3

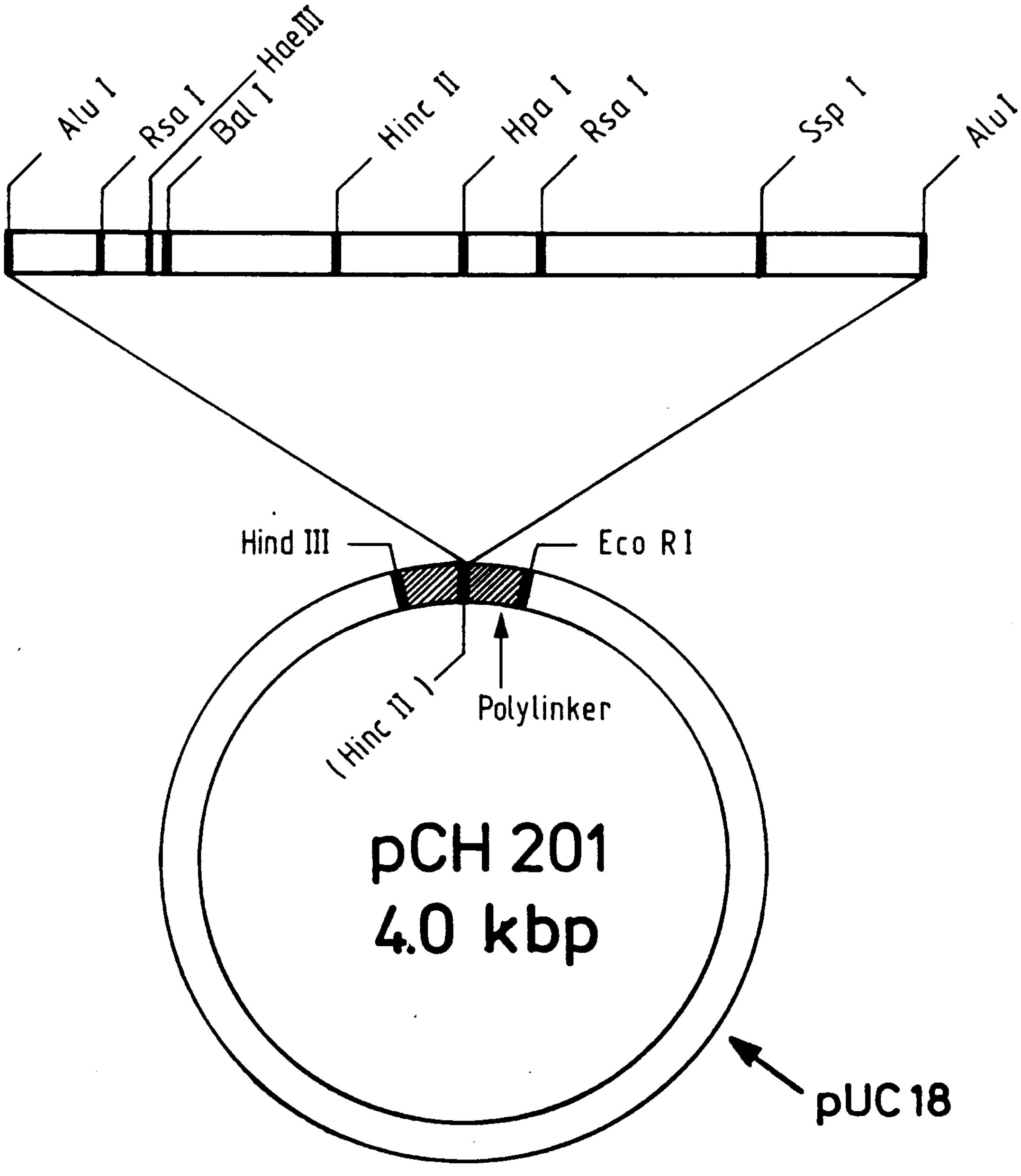


Pat. 1 - 3

Abbildung 4 a

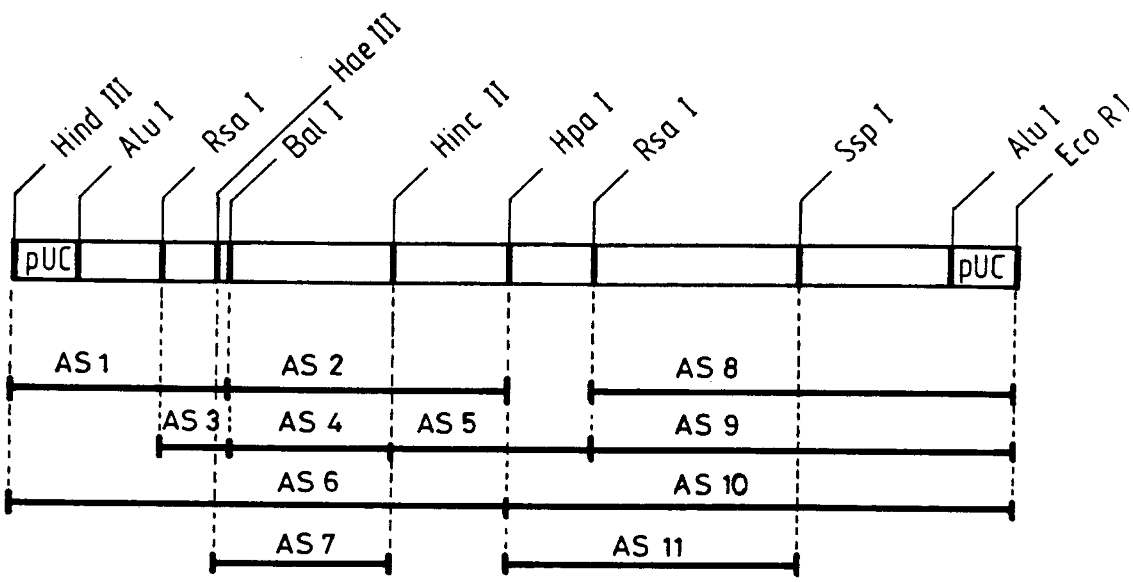


ASx = Subklone nad A-Gen in pUC 18

Abbildung 4 b

5'-CTTACT CCTGCAAGCA ACTCTATGTC
GGTGGAATTA GGCGTAAAAT GACGCATCCT GCACATTAGG
CGTAATTCGA GTGACTTTTC CCCACCATTC GACTATCTTG
TTTAGCATAT AAAACAAATT ACACCGATAA CAGCGAATAT
TACGCTAATG TCGGTTTTAA CGTTAAGCCT GTAAAACGAG
ATGGTAAGAT GAGCGTAATG TTTGATCCAG ACACGGCGAT
TATCCTTTCC CCCCGAAGCC GACGCCGTTA AGCATTGATG
AAAAAGCGTA TTACCGCGAG AAGATAAAAC TGCGTCTAAA
AGAACGTAAT GCGGTGATGG TTGCCCACTA CTATACCGAT
CCCGAAATTC AACAACTGGC AGAAGAAACC GGTGGCTGTA
TTTCTGATTC TCTGGAAATG GCGCGCTTCG GTGCAAAGCA
TCCCGCTTCT ACTTTGTTAG TCGCTGGGGT GAGATTTATG
GGAGAAACCG CCAAAATTCT CAGTCCGGAA AAAACAATTC
TGATGCCGAC ACTTCAGGCT GAATGTTCAC TGGATCTCGG
CTGCCCTGTT GAAGAATTTA ACGCATTTTG CGATGCCCAT
CCCGATCGTA CTGTCGTCGT CTACGCCAAC ACTTCTGCTG
CGGTAAAAGC GCGCGCAGAT TGGGGTGTAA CTTCAAGCAT
TGCCGTCGAA CTTATTGATC ATCTTGATAG TTTGTGTGAA
AAAATCATCT GGGCACCCGA CAAACATCTG GGGCGTTACG
TGCAAAAACA GACGGGTGGA GACATTCTAT GCTGGCAGGG
TGCCTGTATT GTGCATGATG AATTTAAGAC TCAGGCGTTA
ACCCGCTTGC AAGAAGAATA CCCGGATGCT GCCATACTGG
TGCATCCAGA ATCACCACAA GCTATTGTCG ATATGGCGGA
TGCGGTCGGT TCCACCAGTC AACTGATCGC TGCTGCGAAA
ACATTGCCAC ATCAGAGGCT TATTGTGGCA ACCGATCGGG
GTATTTTCTA CAAAATGCAG CAGGCGGTGC CAGATAAAGA
GTTACTGGAA GCACCAACCG CAGGTGAGGG TGCAACCTGC
CGCAGCTGCG CGCATTGTCC GTGGATGGCC ATGAATGCCT
TAGGCCATCG CAGAGGCATT AGAACAGGAA GGAAGCAATC

Abbildung 4 b, Fortsetzung

ACGAGGTTCA TGTTGATGAA AGGCTGCGGA GAAGGGCGCT
GGTGCCGCTC AATCGTATGC TGGATTTTGC GGCTACACTA
CGTGGATAAC GAATAATAAG GCGTAACGTT ACGCTTTGGG
GGAAAGATGG ATTTTTTTAG TGTGCAGAAT ATCCTGGTAC
ATATACCAAT AGGGGCAGGC GGTTATGATC TCTCATGGAT
CGAAGCGGTA GGCACGATCG CCGGGTTGCT GTGTATTGGC
CTTGCCAGTC TGGAGAAGAT CAGCAACTAC TTCTTTGGCC
TGATCAACGT CACCTTGTTT GGCATTATTT TCTTTCAGAT
TCAG-3'

Abbildung 5

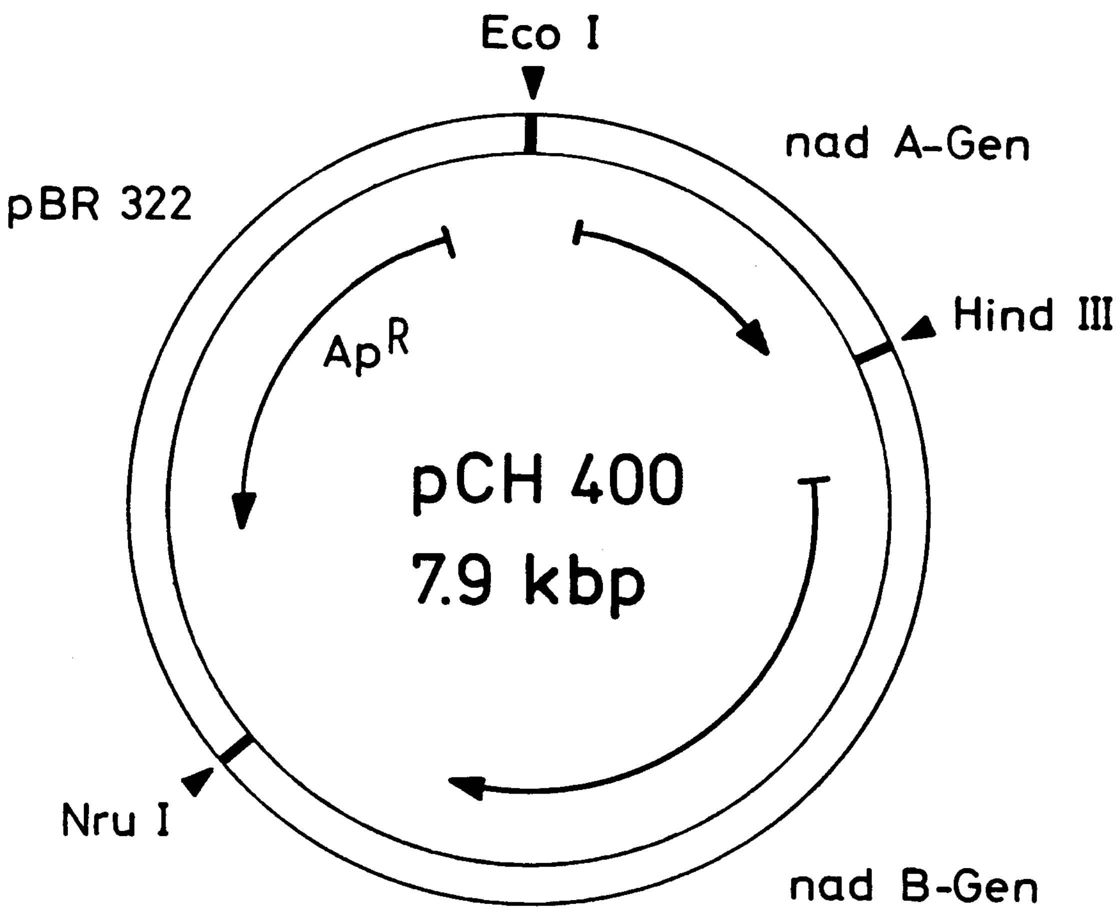


Plasmid pCH 400

Pat. 1 - 5